# EUROPEAN PATENT APPLICATION

(11) **EP 0 532 167 A2**
(43) Date of publication of application: **17.03.1993**
(21) Application number: 92306952.0
(22) Date of filing: 30.07.1992
(51) Int. Cl.: C12N 15/51, A61K 39/29, G01N 33/576, C07K 15/00, C12P 21/08

(54) **Non-A, Non-B Hepatitis virus genome, polynucleotides, polypeptides, antigen, antibody and detection systems**

(30) Priority: 09.08.1991 JP 287402/91; 05.12.1991 JP 360441/91
(71) Applicant: IMMUNO JAPAN INC., Tokyo 167 (JP)
(72) Inventor: Okamoto, Hiroaki, Kawachi-gun; Tochigi-ken 329-04 (JP); Nakamura, Tetsuo, Suginami-ku, Tokyo 167 (JP)
(74) Representative: Arthur, Bryan Edward

(57) **Abstract**

Non-A, non-B hepatitis (NANB hepatitis) virus RNA and its corresponding polypeptide, related antigen, antibody, and detection systems for detecting NANB hepatitis antigen or antibodies.

## Description

### Reference To A Related Application

The present application is a continuation-in-part of our copending U.S. Patent Application Serial No. 07/866,045, filed on April 9, 1992, which is incorporated by reference in its entirety.

### Background of the Invention

The present invention concerns non-A, non-B hepatitis (hereinafter called NANB hepatitis) virus genome, polynucleotides, polypeptides, related antigen, antibody and detection systems for detecting NANB antigens or antibodies.

Viral hepatitis of which DNA and RNA of the causative viruses have been elucidated, and their diagnosis and even prevention in some have been established, are hepatitis A and hepatitis B. The general name NANB hepatitis was given to the other forms of viral hepatitis.

Post-transfusion hepatitis was remarkably reduced after introduction of diagnostic systems for screening hepatitis B in transfusion bloods. However, there are still an estimated 280,000 annual cases of post-transfusion hepatitis caused by NANB hepatitis in Japan.

NANB hepatitis viruses were recently named C,D and E according to their types, and scientists started a world wide effort to conduct research for the causative viruses and subsequent extermination of the causative viruses.

In 1988, Chiron Corp. claimed that they had succeeded in cloning RNA virus genome, which they termed hepatitis C virus (hereinafter called HCV), as the causative agent of NANB hepatitis and reported on its nucleotide sequence (British Patent 2,212,511 which is the equivalent of European Patent Application 0,318,216). HCV (C100-3) antibody detection systems based on the sequence are now being introduced for screening of transfusion bloods and for diagnosis of patients in Japan and in many other countries. The detection systems for the C100-3 antibody have proven their partial association with NANB hepatitis; however, they capture only about 70% of carriers and chronic hepatitis patients, or they fail to detect the antibody in acute phase infection, thus leaving problems yet to be solved even after development of the C100-3 antibody by Chiron Corp.

The course of NANB hepatitis is troublesome and most patients are considered to become carriers, then to develop chronic hepatitis. In addition, most patients with chronic hepatitis develop liver cirrhosis, then hepatocellular carcinoma. It is therefore very imperative to isolate the virus itself and to develop effective diagnostic reagents enabling earlier diagnosis.

The presence of a number of NANB hepatitis which cannot be diagnosed by Chiron's C100-3 antibody detection kits suggests a possibility of a difference in subtype between Chiron's HCV and Japanese NANB hepatitis virus.

In order to develop NANB hepatitis diagnostic kits of more specificity and to develop effective vaccines, it becomes an absolutely important task to analyze each subtype of NANB hepatitis causative virus at its genetic and corresponding amino acid level.

### Summary of the Invention

An object of the present invention is to provide the nucleotide sequence coding for the structural protein of NANB hepatitis virus and, with such information, to analyze amino acids of the protein to locate and provide polypeptides useful as antigen for establishment of detection systems for NANB virus, its related antigens and antibodies.

A further object of the present invention is to locate polynucleotides essential to treatment, prevention and diagnosis, and polypeptides effective as antigens, by isolating NANB hepatitis virus RNA from human and chimpanzee virus carriers, cloning the cDNA covering the whole structural gene of the virus to determine its nucleotide sequence, and studying the amino acid sequence of the cDNA. As a result, the inventors have determined the nucleotides of the whole genome of a strain of NANB virus called HC-J6 and a strain called HC-J8. NANB hepatitis virus genome of HC-J6 and HC-J8 differ from that of Chiron's HCV.

### Brief Description of the Drawings

Figure 1 shows the restriction map and structure of the coding region of NANB hepatitis virus genome (HC-J6) and positions of clones. C, E, NS-1, NS-2, NS-3, NS-4 and NS-5 are the abbreviation of core, envelope, non-structure-1, -2, -3, -4 and -5.

Figures 2 to 4 show method of determination of the nucleotide sequence of 5' terminus of NANB hepatitis virus genome of strains HC-J1, HC-J4 and HC-J6 respectively.

Figure 5 shows the method of determination of the nucleotide sequence of 3' terminus of HC-J6 genome. Solid lines show nucleotide sequences determined by clones from libraries of bacteriophage lambda gt10, and broken lines show nucleotide sequences determined by clones obtained by PCR.

Figure 6 shows the structure of coding region of NANB hepatitis virus genome (HC-J8) and positions of clones. Regions a to n indicate positions of amplification by PCR.

### Detailed Description of the Invention

The present invention provides NANB hepatitis virus genome RNA for strain HC-J6 (sequence list 1) consisting of 340 nucleotides on the 5' terminus that follow an open reading frame consisting of 9099 nucleotides coding for the structural protein and non-structural protein that follow a noncoding region consisting of 150 nucleotides containing an U-stretch consisting of 108 uracils on the 3' terminus of NANB hepatitis virus, and NANB hepatitis virus genome having substantially the nucleotide sequence of sequence list 1.

The present invention provides polynucleotide N-9589 (strain HC-J6) comprising the DNA nucleotide sequence of sequence list 2; cDNA clone J6-ø81 comprising the nucleotide sequence of sequence list 3; cDNA clone J6-ø8 comprising the nucleotide sequence of sequence list 4; and NANB hepatitis virus polynucleotides having substantially the sequence of nucleotides of NANB hepatitis virus nucleotides shown in sequence lists 2 through 4.

The invention provides polypeptide coded for by genome or polynucleotide of HC-J6 above, polypeptide P-J6-3033, comprising the polypeptide sequence of sequence list 5, polypeptides produced by using recombinant genome, recombinant polynucleotides and recombinant cDNA of whole or a part of cDNA above, and polyclonal or monoclonal antibodies against the polypeptides described above.

The present invention also provides NANB hepatitis virus genome for strain HC-J8 comprising sequence list 6, NANB hepatitis virus RNA consisting of noncoding region consisting of 341 nucleotides on 5' terminus followed by an open reading frame consisting of 9099 nucleotides coding for the structural protein and non-structural protein followed by a noncoding region consisting of 71 nucleotides containing an U-stretch consisting of 30 uracils on 3' terminus of NANB hepatitis virus comprising sequence list 6, and NANB hepatitis virus genome having substantially the nucleotide sequence of sequence list 6.

The present invention provides polynucleotide N-9511 for strain HC-J8 comprising the DNA nucleotide sequence of sequence list 7 and NANB hepatitis virus polynucleotide having substantially the sequence of nucleotides of NANB hepatitis virus nucleotides comprising sequence list 7.

The invention provides polypeptide coded for by genome or polynucleotide of HC-J8 above, polypeptide P-J8-3033, comprising the polypeptide sequence of sequence list 8 and polypeptide P-J8-3033-2 comprising the polypeptide sequence of sequence list 9, polypeptides produced by using recombinant genome, recombinant polynucleotides and recombinant cDNA of whole or a part of cDNA above, and polyclonal or monoclonal antibodies against the polypeptides described above.

The present invention, furthermore, provides NANB hepatitis diagnostic system using polypeptides or antibodies described above.

In the method described below, NANB hepatitis virus RNA of the present invention was obtained and its nucleotide sequence was determined.

Plasma samples (HC-J1, HC-J4, HC-J6 and HC-J8) were obtained from human and chimpanzee. HC-J1, HC-J6 and HC-J8 were obtained from Japanese blood donors who had tested positive for HCV antibody. HC-J4 was obtained from the chimpanzee subjected to the challenge test but was negative for Chiron's C100-3 antibody previously mentioned.

RNA was isolated from each of the plasma samples. Following the study of 5' terminus of approximately 2,500 nucleotides and 3' terminus of approximately 1,100 nucleotides disclosed in Japanese patent application No. 196175/91, the inventors have completed the study of the region coding for non-structural protein of strain HC-J6 and the study of the full length sequence of 9,589 nucleotides of HC-J6 genome RNA and have completed the study of the region coding for non-structural protein of strain HC-J8 and the study of the full length sequence of 9,589 nucleotides of HC-J8 genome RNA.

As described in the Example below, strain HC-J6 had a 5' noncoding region consisting of 340 nucleotides, and strain HC-J8 had a 5' noncoding region consisting of 341 nucleotides, followed by region coding for structural protein and region coding for non-structural protein.

Concerning the 3' terminus, strain HC-J6 was found to have a region consisting of 150 nucleotides containing an U-stretch consisting of 108 uracils following after the region coding for non-structural protein and strain HC-J8 was found to have a region consisting of 71 nucleotides containing an U-stretch consisting of 30 uracils following after the region coding for non-structural protein.

The coding region starting with adenine (341st nucleotide from the 5' terminus for strain HC-J6 and 342nd nucleotide from the 5' terminus for strain HC-J8) was found to have a long *Open Reading Frame* consisting of 9099 nucleotides which codes for 3033 amino acids. HCV or hepatitis C virus is supposed to be closely allied to flavivirus in regard to its genetic structure. The coding of the NANB hepatitis virus genome of the present invention was considered to be consisting of regions named C (core), E (envelope), NS-1 (non-structural-1), NS-2 (non-structural-2), NS-3 (non-structural-3), NS-4 (non-structural-4) and NS-5 (non-structural-5).

As compared with the sequence of HCV disclosed in the European Patent Application by Chiron Corp. (Publication No. 388,232), homology of sequences of the strain HC-J6 was 67.9% for the full nucleotide sequence and 72.3% for the full amino acid sequence, and homology of sequences of the strain HC-J8 was 66.4% for the full nucleotide sequence and 71.0% for the full amino acid sequence.

From an examination of homology for regions, the homology of nucleotide sequences (strain HC-J6) of the 5' terminal noncoding region was 94.4% and that of the amino acid sequences of the C region was 90.1%, showing comparatively high homology; on the other hand, concerning lower stream than envelope, homologies of amino acid sequence were found to be as low as 60.4% for E, 71.1% for NS-1, 57.8% for NS-2, 81.1% for NS-3, 73.1% for NS-4, and 69.9% for NS-5. As a result, HC-J6 strain was found to be significantly different from HCV strain found by Chiron Corp.

From an examination of homology for regions, the homology of nucleotide sequences (strain HC-J8) of the 5' terminal noncoding region was 93.8% and that of the amino acid sequences of the C region was 90.1%, showing comparatively high homology; on the other hand, concerning lower stream than envelope, homologies of amino acid sequence were found to be as low as 54.7% for E, 73.1% for NS-1, 55.6% for NS-2, 81.3% for NS-3, 72.1% for NS-4, 67.3% for NS-5, and 25.9% for 3' terminal noncoding region. As a result, HC-J8 strain was found to be significantly different from HCV strain found by Chiron Corp.

From the comparison of amino acid sequence of HC-J6 strain with strain HC-J1 (American type) and strain HC-J4 (Japanese type) disclosed by the inventors (Japan. J. Exp. Med. (1990), 60: 167-177), homology in the core region was more than 90% for each strain while that in the envelope region was 60.9% for HC-J1 and 53.1% for HC-J4. Thus, in the present invention, strain HC-J6 was found to be a different type of virus than strains HC-J1 or HC-J4.

From the comparison of amino acid sequence of HC-J8 strain with strain HC-J1 (type I) and strain HC-J4 (type II), homology of approximately 3,000 nucleotides of 5' terminus was 70.1% for HC-J1 and 67.1% for HC-J4, and from the comparison of all nucleotides with HC-J6 (type III) genome homology was as low as 76.9%. On the other hand, HC-J8 showed high homology with strain HC-J7 (type IV) disclosed in Japanese patent application 196175/91 as 93.1% for approximately 3,000 nucleotides of 5' terminus.

Nucleotides among stains assumed to belong to same type were supposed to show high homology. For example, homology of 95.6% for approximately 3,000 nucleotides of 5' terminus between HCV disclosed by Chiron Corp. and HC-J1 appears to show that they should be classified into type I. On the other hand, low homology of HC-J8 with HCV, HC-J1, HC-J4 and HC-J6 appeared to show that it was not to be classified into type I, II or III, but into type IV (the same as HC-J7).

Strain HC-J8 has some mutations in the nucleotides as shown in sequence lists 6 and 7 by symbols M, R, W, S, Y, K and B. It also can be easily understood that it has some mutations of amino acids from comparison of sequences in sequences lists 8 and 9. Mutation of nucleotides was observed up to approximately 1.4% in the whole genome and that of amino acids was observed up to approximately 1.7% in whole ORF. Thus the present invention includes genomes, polynucleotides and polypeptides of strain HC-J8 having some mutations.

In addition, envelope (E) region (576 nucleotides/192 amino acids of amino acids 192-383) and NS-1 region (1050 nucleotides/350 amino acids of amino acids 384-733) having many mutations in HC-J8 are called hyper-variable region since mutations were observed as 20 nucleotides/7 amino acids (3.47%/3.64%) in E region and 37 nucleotides/19 amino acids (3.52%/5.42%) in NS-1 region. According to these findings, the present invention can be recognized to include genomes and polypeptides coded for by the genomes of strain HC-J8 having mutations of 3.5% to 5.5% in those regions.

The genome, polynucleotide, and cDNA clones of the present invention can be used as material to produce peptides of the invention by integration into a host genome, e.g. *E. coli* or *Bacillus,* by means of known genetic engineering techniques.

Polypeptides of the invention are useful as material for diagnostic agents to detect NANB hepatitis antibodies with high specificity and as material to produce polyclonal and monoclonal antibodies by known techniques.

Polyclonal and monoclonal antibodies of the invention are useful as materials for diagnostic agents to detect NANB hepatitis antigens with high specificity.

A detection system using each polypeptide of the present invention or polypeptide with partial replacement of amino acids, and a detection system using monoclonal or polyclonal antibodies to such polypeptides, are useful as diagnostic agents of NANB hepatitis with high specificity and are effective to screen out NANB hepatitis virus from transfusion bloods or blood derivatives. The polypeptides, or antibodies to such polypeptides, can be used as a material for a vaccine against NANB hepatitis virus.

It is well known in the art that one or more nucleotides in a DNA sequence can be replaced by other nucleotides in order to produce the same protein. The present invention also concerns such nucleotide substitutions which yield DNA sequences which code for polypeptides as described above. It is also well known in the art that one or more amino acids in an amino acid sequence can be replaced by equivalent other amino acids, as demonstrated by U.S. Patent No. 4,737,487 which is incorporated by reference, in order to produce an analog of the amino acid sequence. Any analogs of the polypeptides of the present invention involving amino acid deletions, amino acid replacements, such as replacements by other amino acids, or by isosteres (modified amino acids that bear close structural and spatial similarity to protein amino acids), amino acid additions, or isosteres additions can be utilized, so long as the sequences elicit antibodies recognizing NANB antigens.

Examples of application of this invention are shown below, however, the invention shall in no way be limited to those examples.

### Examples

The 5' terminal nucleotide sequence and amino acid sequence of NANB hepatitis virus genome were determined in the following way:

### (1) Isolation of RNA

RNA of the sample (HC-J1, HC-J6, HC-J8) from plasma of Japanese blood donor testing positive for HCV (C100-3) antibody (by Ortho HCV Ab ELISA, Ortho Diagnostic System, Tokyo), and that of the sample (HC-J4) from the chimpanzee challenged with NANB hepatitis for infectivity and negative for HCV antibody were isolated in the following method:

Each plasma sample was added with Tris chloride buffer (10 mM, pH 8.0) and centrifuged at 68 x 10³ rpm for 1 hour. Its precipitate was suspended in Tris chloride buffer (50 mM, pH 8.0) containing 200 mM NaCl, 10 mM EDTA, 2% (w/v) sodium dodecyl sulfate (SDS), and proteinase K 1 mg/ml, incubated at 60°C for 1 hour, then their nucleic acids were extracted by phenol/chloroform and precipitated by ethanol to obtain RNA.

### (2) HC-J1 and HC-J8 cDNA Synthesis

After heating the RNA isolated from HC-J1 or HC-J8 plasma at 70°C for 1 minute, this was used as a template; 10 units of reverse transcriptase (cDNA Synthesis System Plus, Amersham Japan) and 20 pmol of oligonucleotide primer (20 mer) were added and incubated at 42°C for 1.5 hours to obtain cDNA. Primer #8 (5'- GATGCTTGCGGAAGCAATCA - 3') was prepared by referring to the basic sequence shown in European Patent Application No. 88310922.5, which is relied on and incorporated herein by reference.

### (3) cDNA Was Amplified by the following Polymerase Chain Reaction (PCR)

cDNA was amplified for 35 cycles according to Saiki's method (Science (1988) 239: 487-491) using Gene Amp DNA Amplifier Reagent (Perkin-Elmer.Cetus) on a DNA Thermal Cycler (Perkin-Elmer.Cetus).

For cDNA synthesis and for PCR for HC-J8, synthesized primers disclosed in Japanese patent application 153402/90 and those based on HC-J1, HC-J4 and HC-J6 genomes disclosed in Japanese patent applications 196175/91 and below were utilized.

### (4) Determination of 5' Terminal Nucleotide Sequence of HC-J1 and HC-J4 by Assembling cDNA Clones

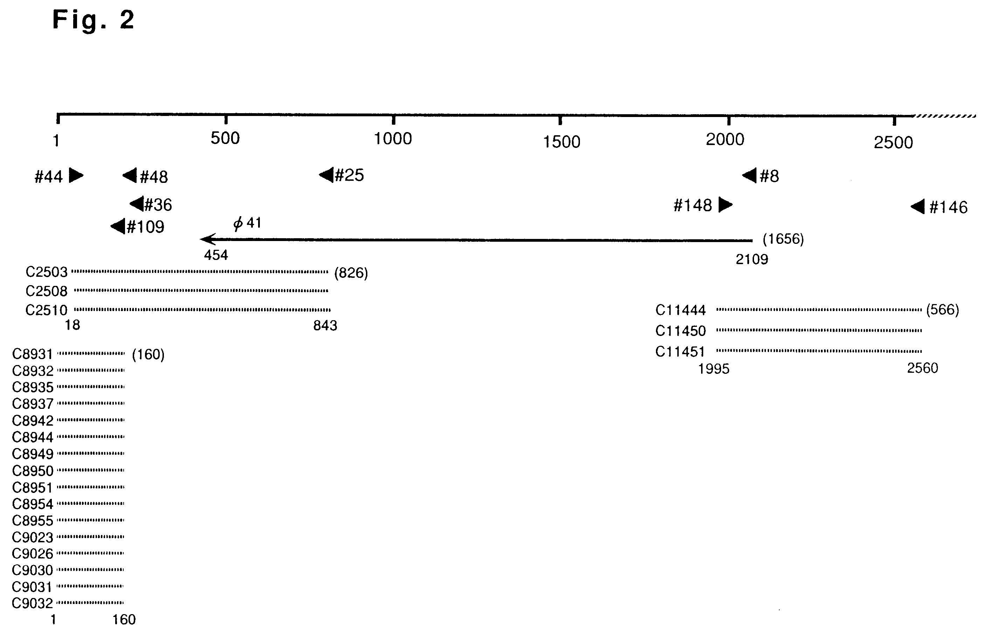

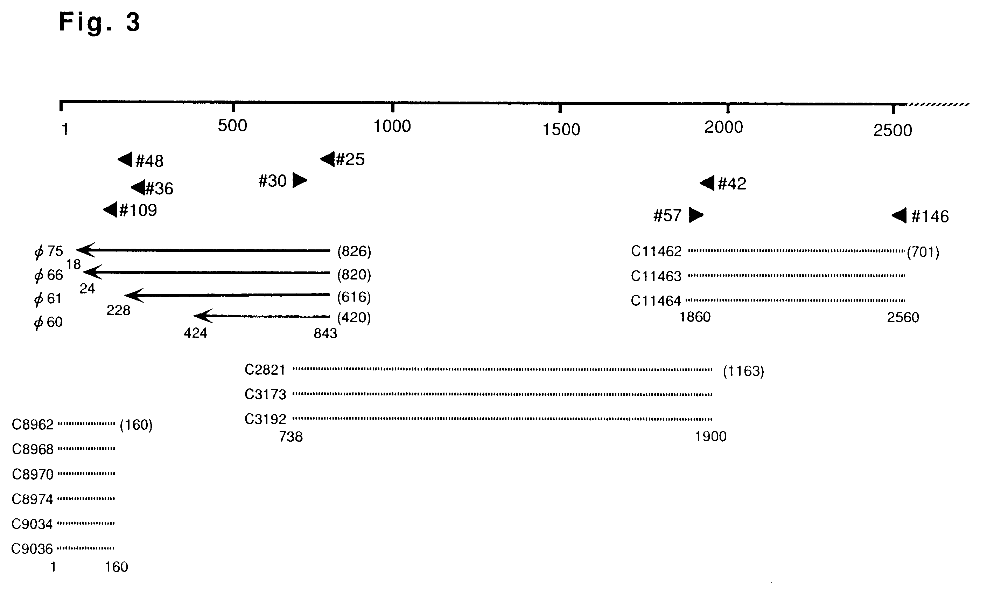

As shown in Figures 2 and 3, nucleotide sequences of 5' termini of the genomes of strains HC-J1 and HC-J4 were determined by combined analysis of clones obtained from the cDNA library constructed in bacteriophage λgt10 and clones obtained by amplification of HCV specific cDNA by PCR.

Figures 2 and 3 show 5' termini of NANB hepatitis virus genome together with cleavage site by restriction endonuclease and sequence of primers used. In the figures, solid lines are nucleotide sequences determined by clones from bacteriophage λgt10 library while dotted lines show sequences determined by clones obtained by PCR.

A 1656 nucleotide sequence of HC-J1 spanning nt454-2109 was determined by clone ø41 which was obtained by inserting the cDNA synthesized with the primer #8 into λgt10 phage vector (Amersham).

Another primer #25 (5'- TCCCTGTTGCATAGTTCACG -3') corresponding to nt824-843 was synthesized based on the ø41 sequence, and four clones (ø60, ø61, ø66 and ø75) were obtained to cover the upstream sequence nt18-843.

### (5) Determination of 5' Terminal Nucleotide Sequence of HC-J6.

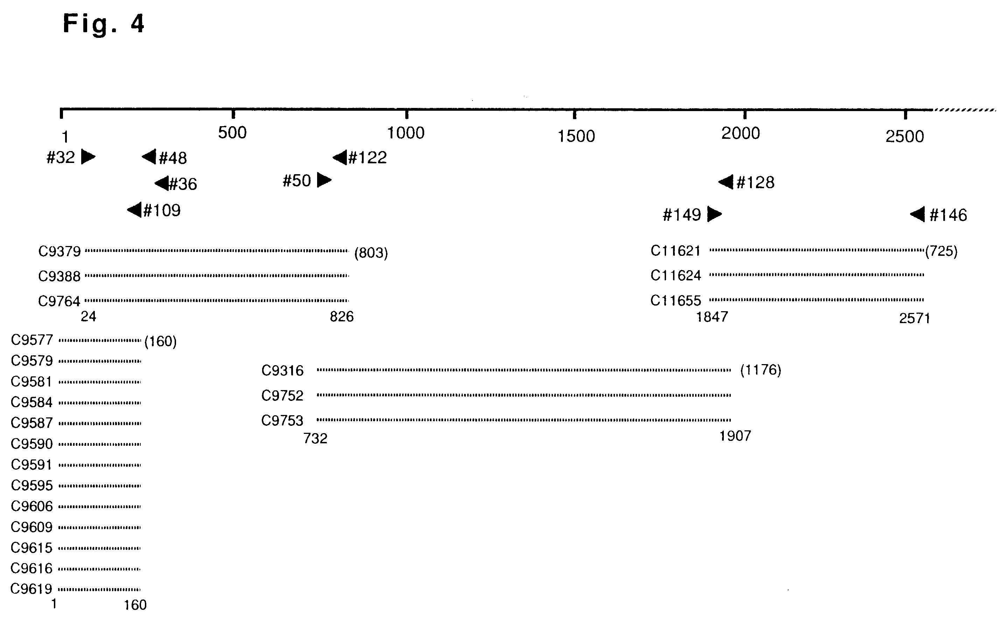

The nucleotide sequence of the 5' terminus of strain HC-J6 was determined from analysis of clones obtained by PCR amplification as shown in Figure 4.

Isolation of RNA from HC-J6 and determination of its sequence was made in the same manner as described in (2) above. Sequences in the range of nt24-2551 of the RNA were determined from consensus sequence of respective clones obtained by amplification by PCR using each pair of primers based on nucleotide sequence of HC-J4. 
nt24-826

#32 (5'-ACTCCACCATAGATCACTCC-3')

#122 (5'-AGGTTCCCTGTTGCATAATT-3')

Clones: C9397, C9388, C9764

nt732-1907

#50 (5'-GCCGACCTCATGGGTACAT-3')

#128 (5'-TCGGTCGTGCCCACTACCAC-3')
 
Clones: C9316,C9752,C9753

nt1847-2571

#149 (5'-TCTGTGTGTGGCCCAGTGTA-3')

#146 (5'-AGTAGCATCATCCACAAGCA-3')

Clones: C11621,C11624,C11655
In order to determine further upstream of the 5' terminus, antisense primer #36 (5'- AACACTACTCGGCTAGCAGT -3') corresponding to nt246-265, followed by dAs were added to 5' terminus of cDNA using terminal deoxynucleotidyl transferase, and one-sided PCR amplification was made twice as described below.

cDNA was amplified for 35 cycles as first stage PCR using oligo dT primer (20-mer) and antisense primer #48 (5'-GTTGATCCAAGAAAGGACCC -3') of nt188-207, followed by the second stage of PCR by 30 cycle amplification using the first PCR product as a template, oligo dT primer (20 -mer) and antisense primer #109 (21-mer; 5'-ACCGGATCCGCAGACCACTAT-3') corresponding to nt140 to 160. The obtained PCR product was subcloned to M13 phage vector.

Nucleotide sequence from nt1 to 23 was determined from consensus sequence of 13 isolated clones C9577, C9579, C9581, C9587, C9590, C9591, C9595, C9606, C9609, C9615, C9616 and C9619 obtained above which were considered having complete 5' terminus.

### (6) Determination of nucleotide sequence of HC-J6 middle region.

cDNA library was constructed with using λgt10 according to the method described in (2) above from 100ml of HC-J6 plasma as a starting materials. Primers #162 and #81 were prepared for synthesis by referring to the basic sequence shown in the European Patent Application Publication No. 318,216. Clones were selected by plaque hybridization.

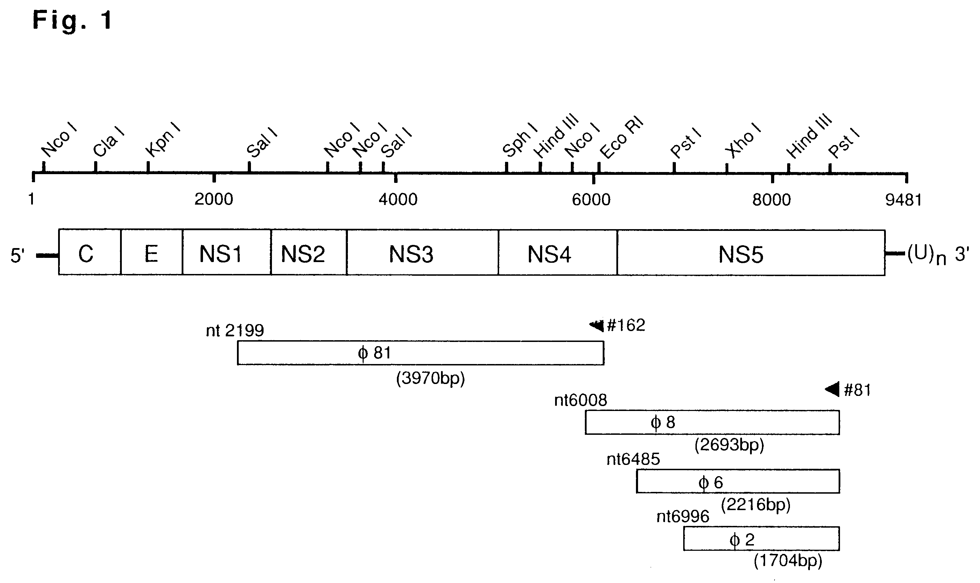

Nucleotide sequence from 2552 to 8700 was determined from consensus sequence of four obtained cDNA clones ø2 (nt6996 to 8700), ø6(nt6485 to 8700), ø8(nt6008 to 8700) and ø81 (nt2199 to 6168) as shown in Figure 1. Clones ø81 and ø8 were found to have nucleotide sequences shown in sequence lists 3 and 4 respectively.

### (7) Determination of 3' terminal nucleotide sequence of HC-J6 strain.

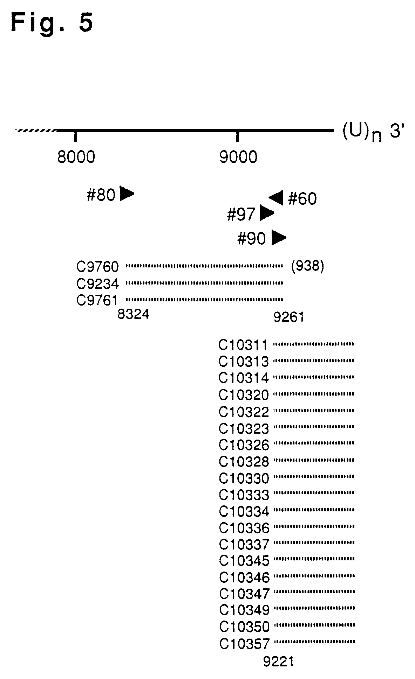

As shown in Figure 5, the nucleotide sequence of the 3' terminus of HC-J6 genome was determined by analysis of clones obtained by amplification of HCV specific cDNA by PCR.

Nucleotide sequence of HC-J6 from nt8701 to 9241 was determined from consensus sequence of three clones consisting of 938 nucleotides, C9760, C9234 and C9761, obtained by amplification of sample using primer #80 (5'-GACACCCGCTGTTTTGACTC-3') and #60 (5'-GTTCTTACTGCCCAGTTGAA-3').

Nucleotide sequence of 3' terminus down stream from nt9242 was determined in the method described below.

Isolation of RNA from HC-J6 was made in the same manner as described in (1) above. The obtained RNA was added poly (A) to its 3' terminus using poly (A) polymerase and cDNA was synthesized using oligo (dT)₂₀ as a primer, and obtained cDNA was provided to PCR as a template.

First PCR product was made with using #97 (5'-AGTCAGGGCGTCCCTCATCT-3') as a sense primer and oligo (dT)₂₀ as an antisense primer. Second PCR product was made with using #90 (5'-GCCGTTTGCGGCCGATATCT-3') corresponding to downstream sequence of #97 as a sense primer, and oligo (dT)₂₀ as an antisense primer as well as first PCR product. PCR product obtained by two step amplification was smoothened on both ends by treatment with T₄DNA polymerase, followed by phosphorylation of 5'terminus by T₄ polynucleotide kinase. The obtained product was subcloned into Hinc II position of M13mp19 phage vector.

Nucleotide sequence of 3' terminus was determined from consensus sequence of 19 obtained clones, C10311, C10313, C10314, C10320, C10322, C10323, C10326, C10328, C10330, C10333, C10334, C10336, C10337, C10345, C10346, C10347, C10349, C10350 and C10357.

As a result, the nucleotide sequence of cDNA to HC-J6 genome RNA was determined as shown in sequence list 2, and full sequence of genome RNA was determined as shown in sequence list 1.

### (8) Determination of amino acid sequences.

According to the nucleotide sequence of the genome of strain HC-J6, determination was made of sequence of coded region starting with ATG. As a result, HC-J6 genome was found to have a long *Open Reading Frame* coding for polypeptide precursor consisting of 3033 amino acid residues.

### (9) Determination of 5' terminal nucleotide sequence of HC-J8

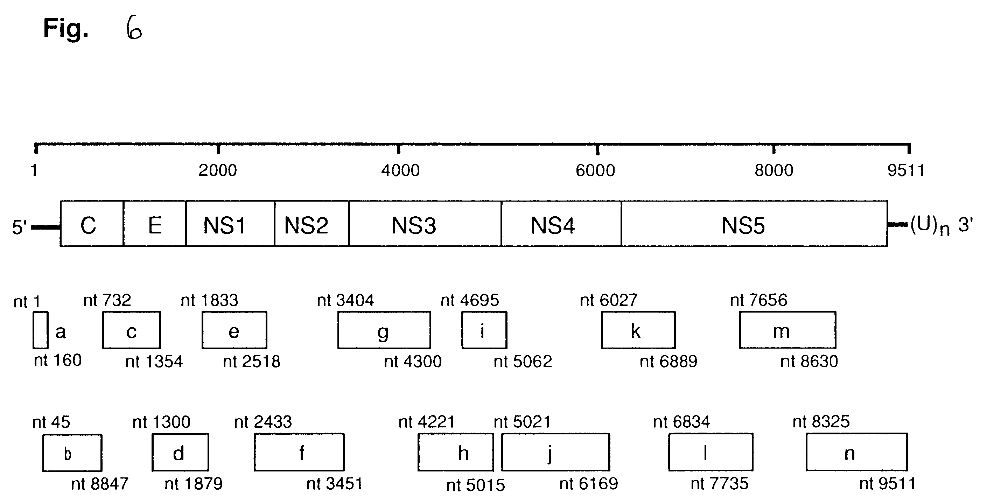

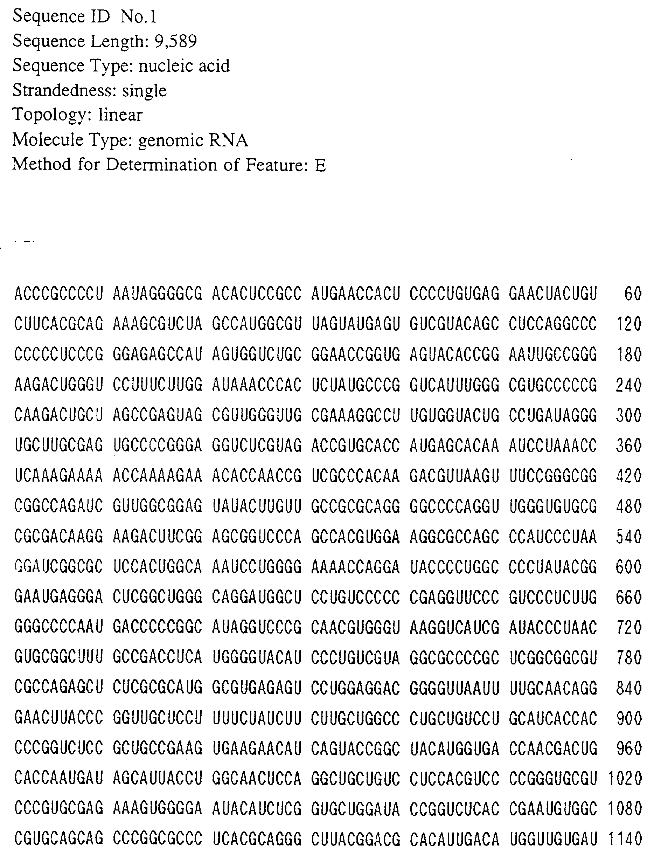

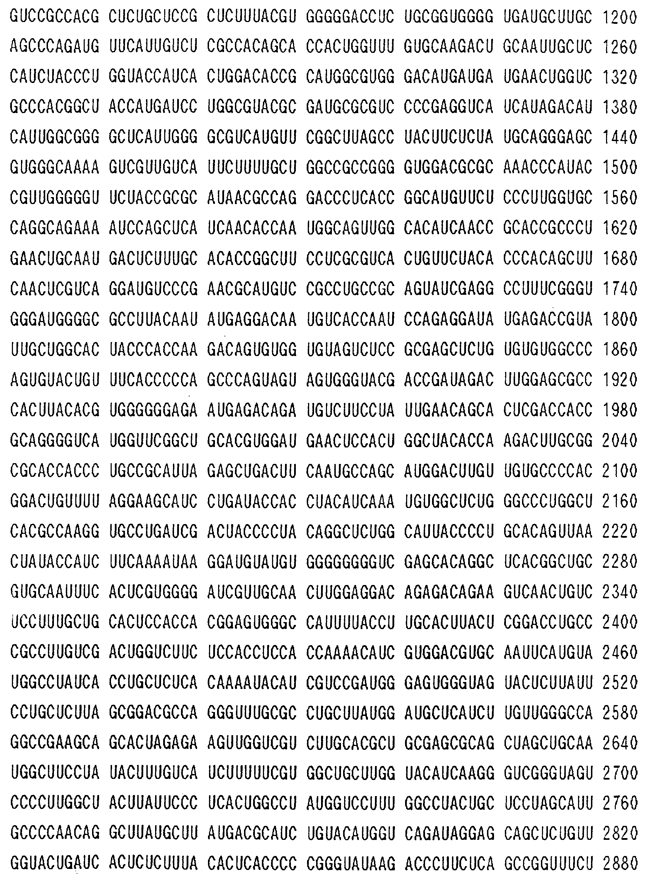

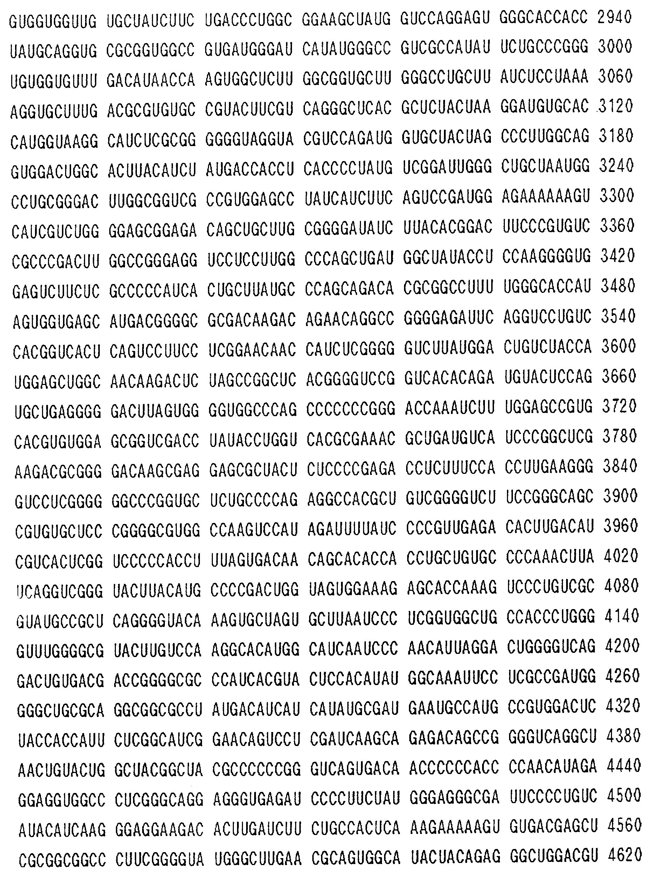

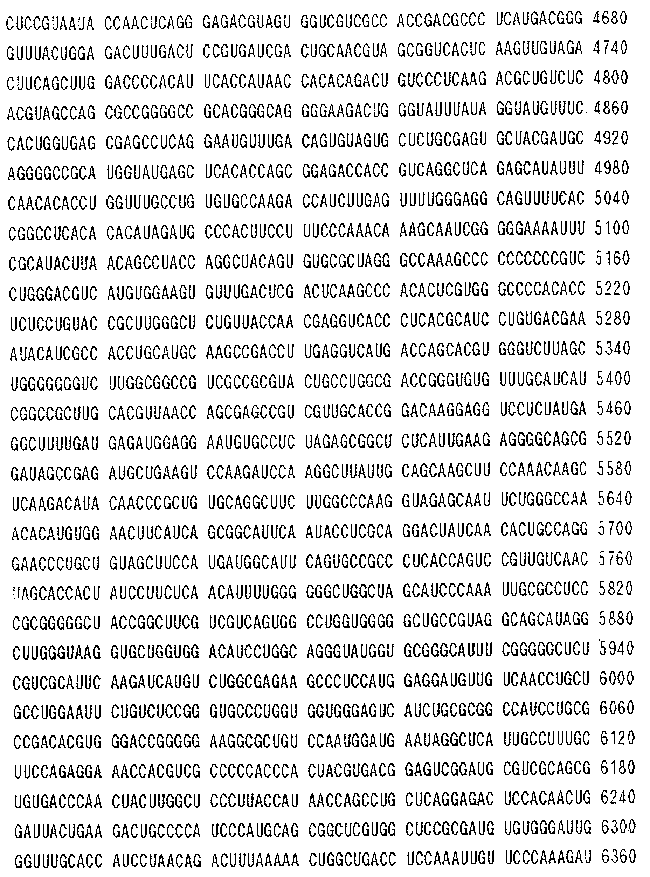

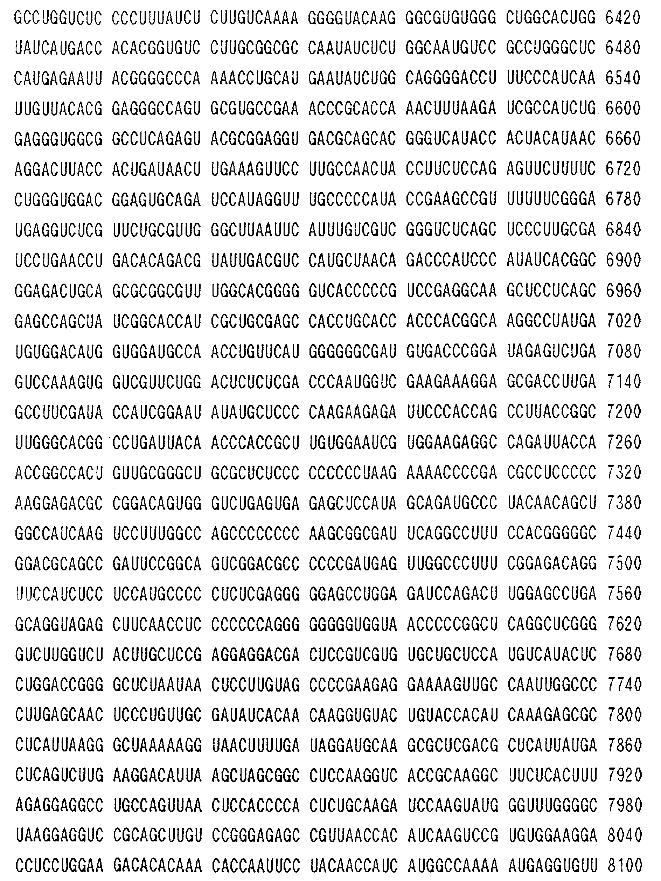

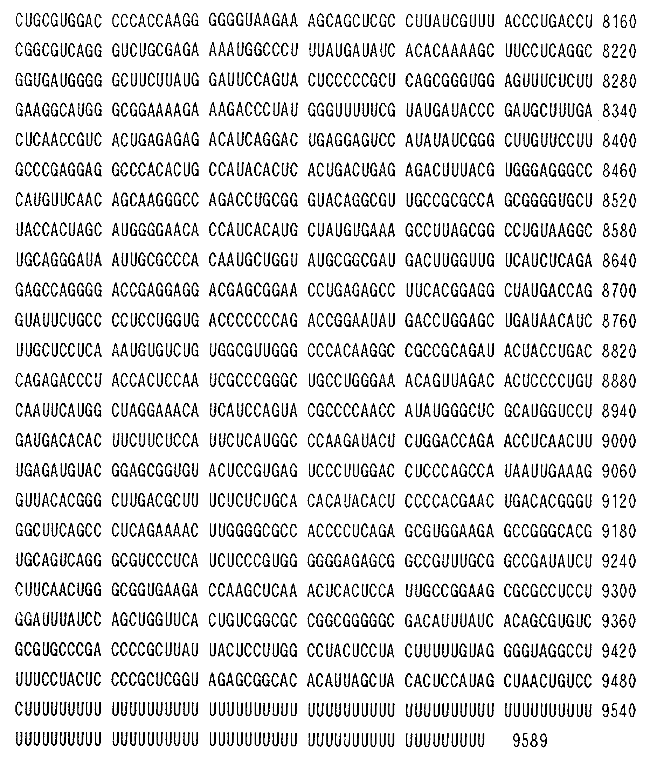

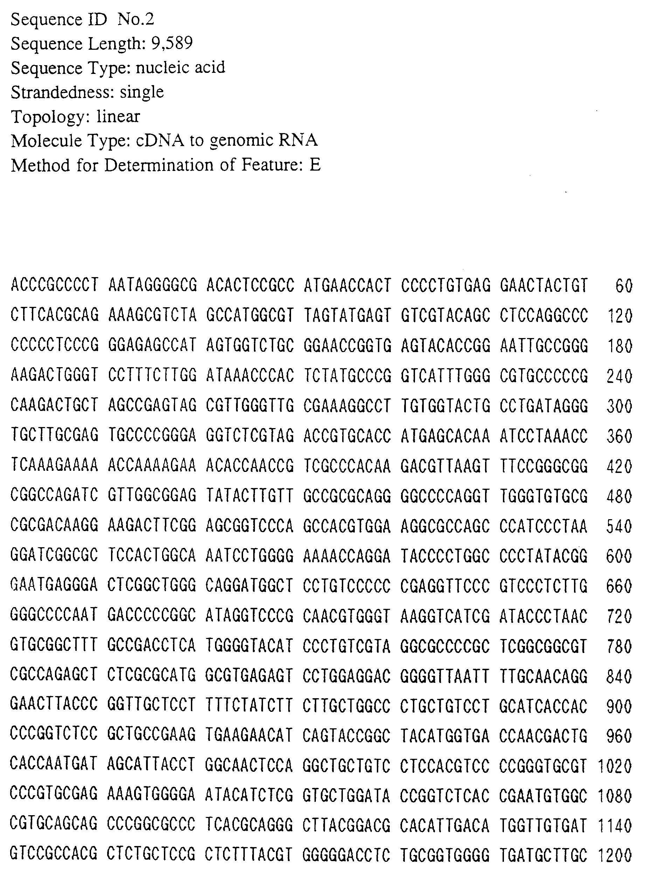

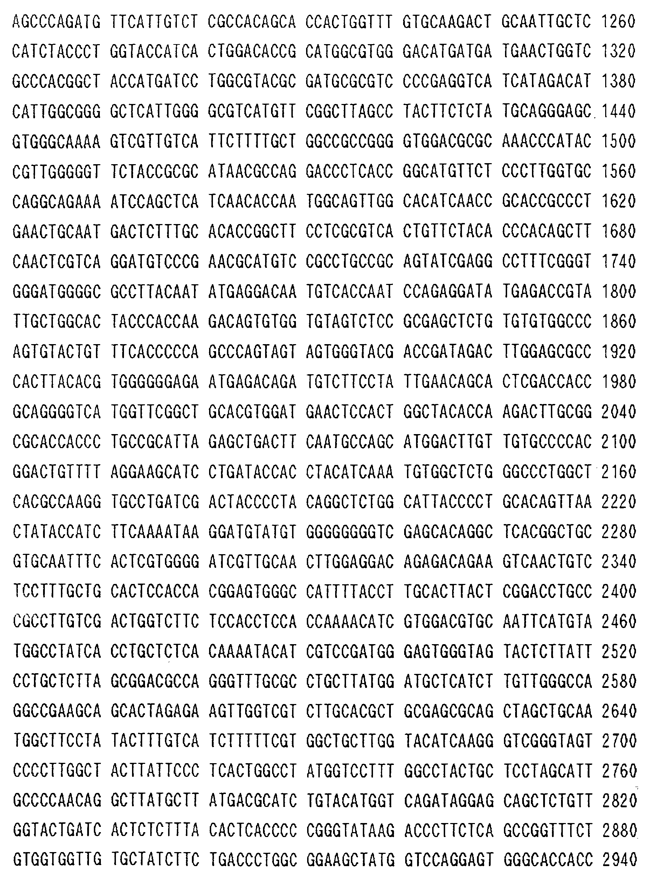

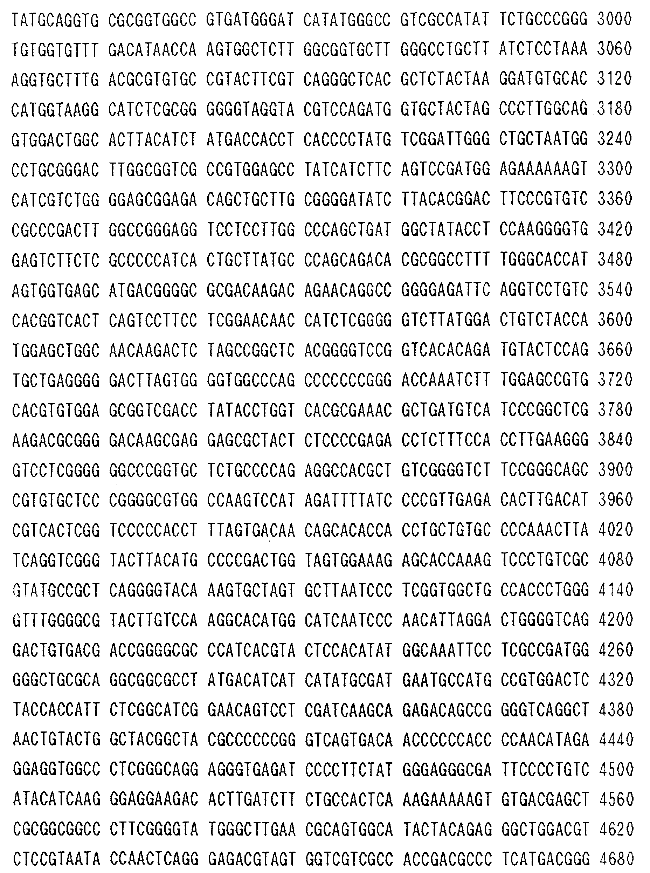

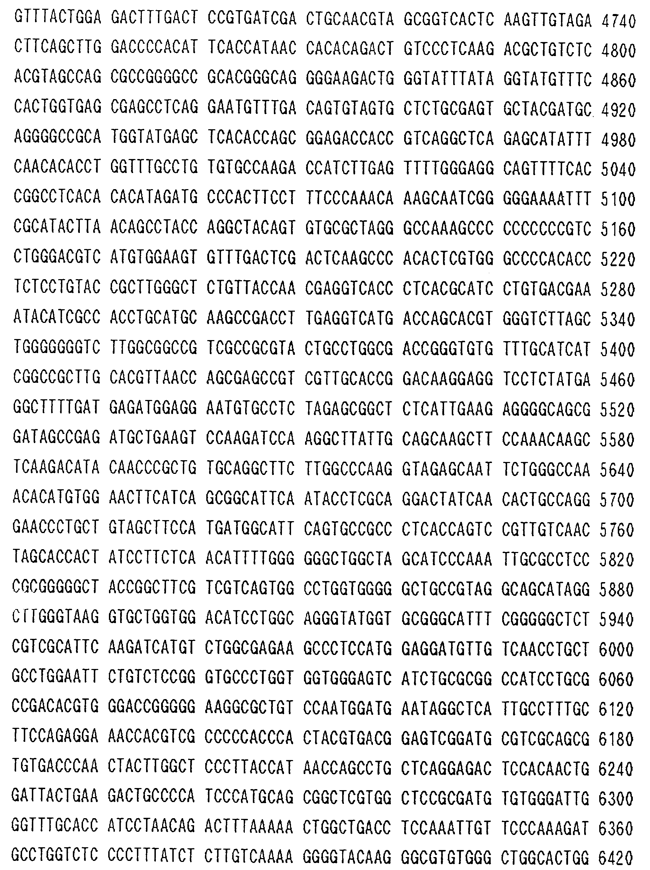

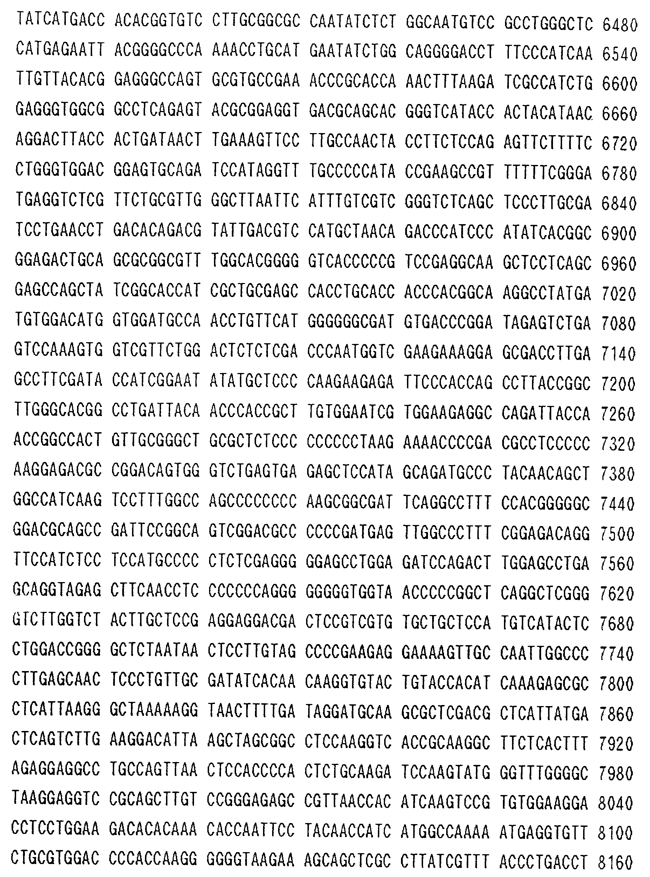

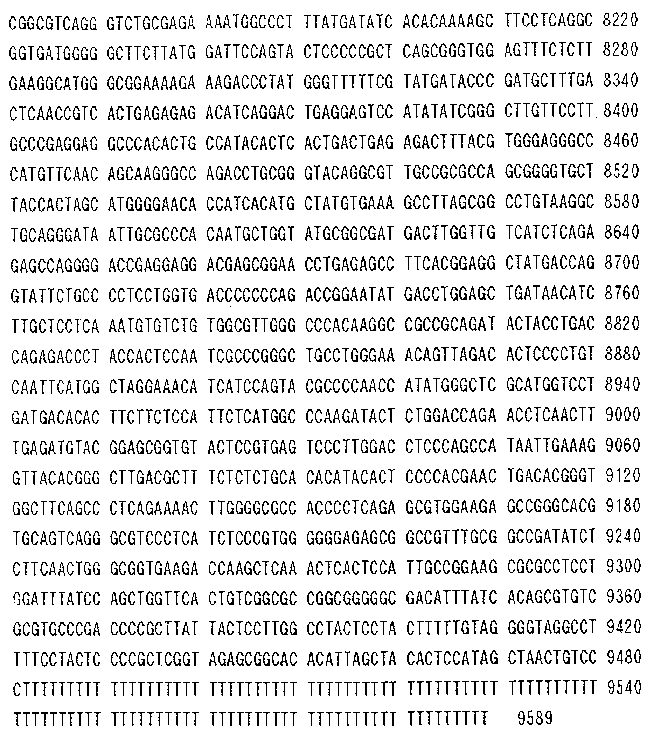

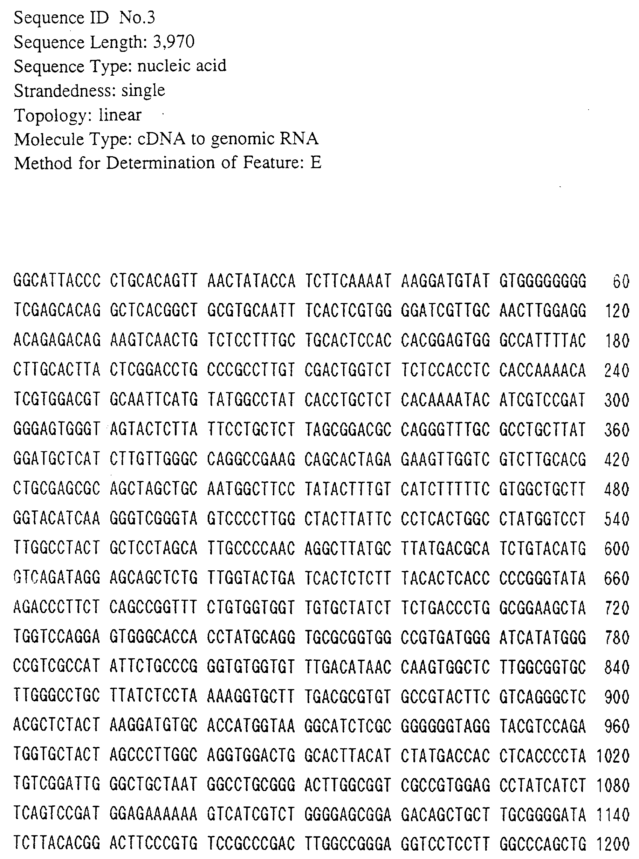

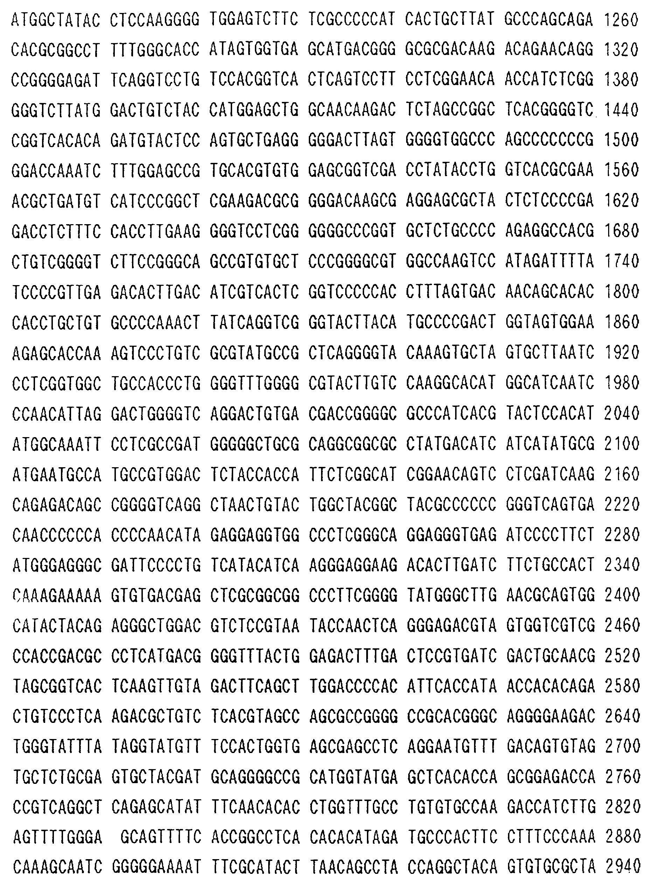

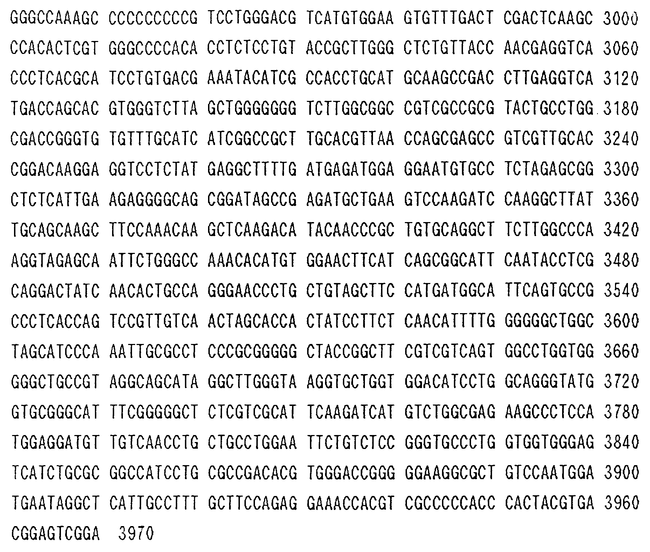

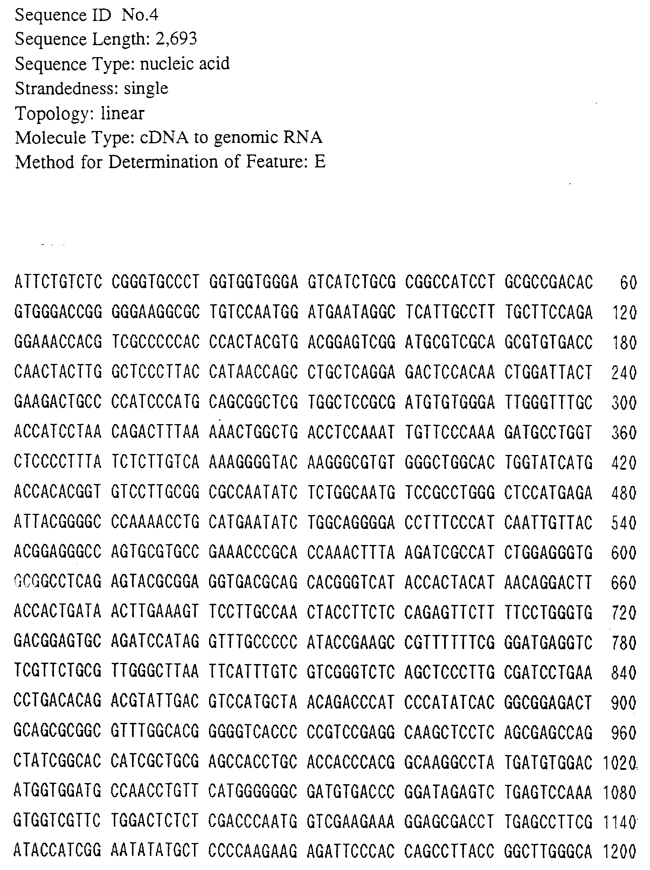

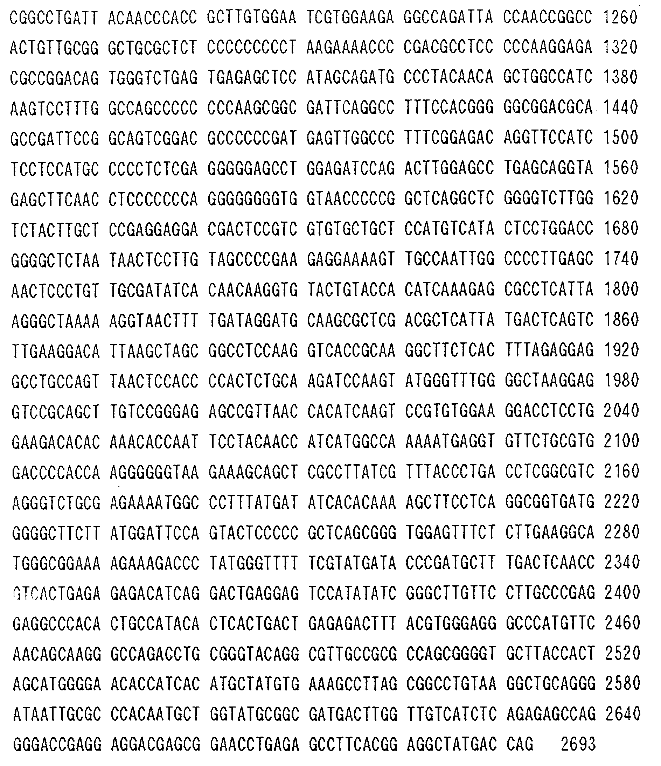

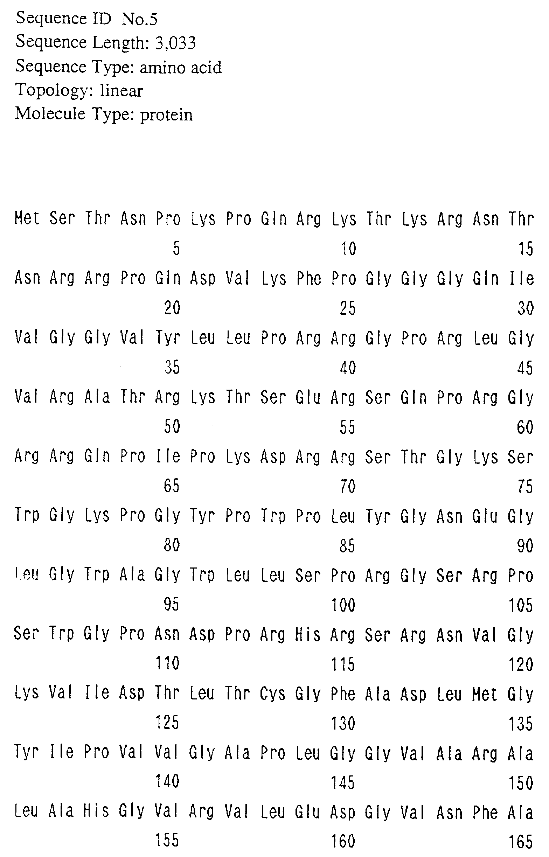

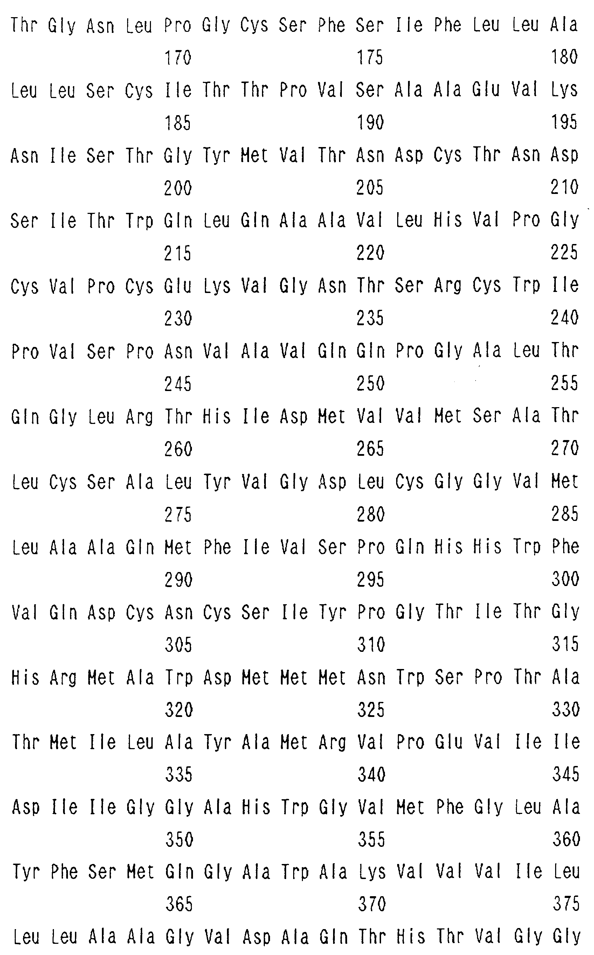

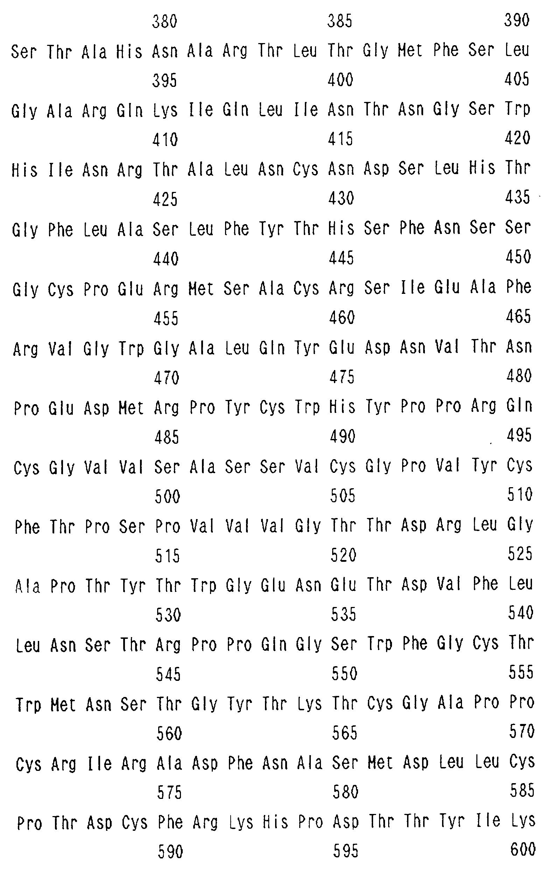

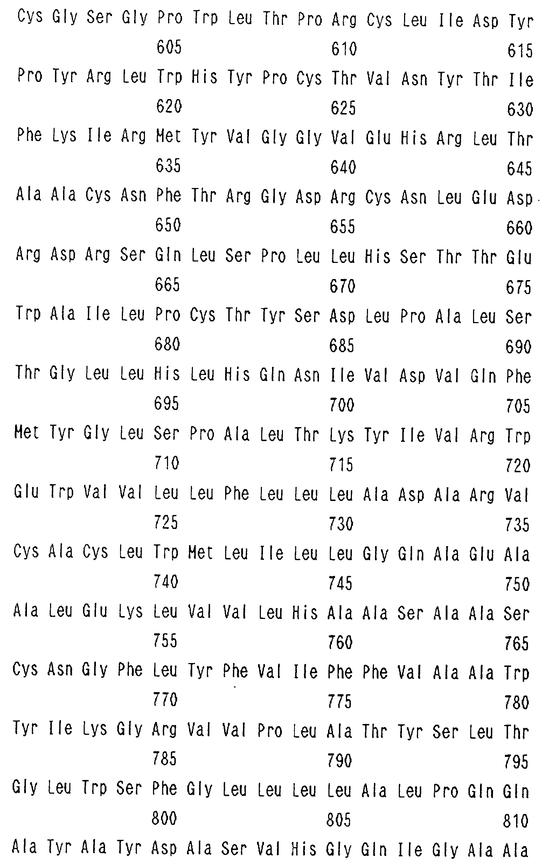

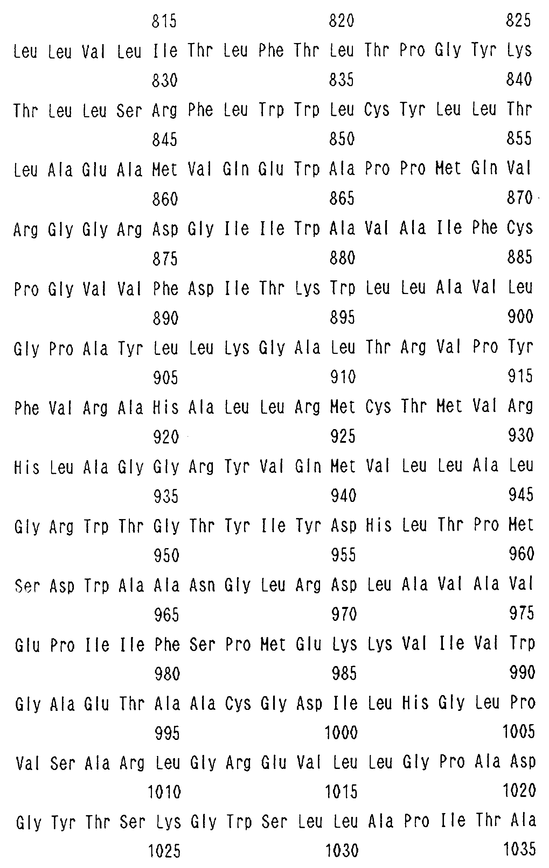

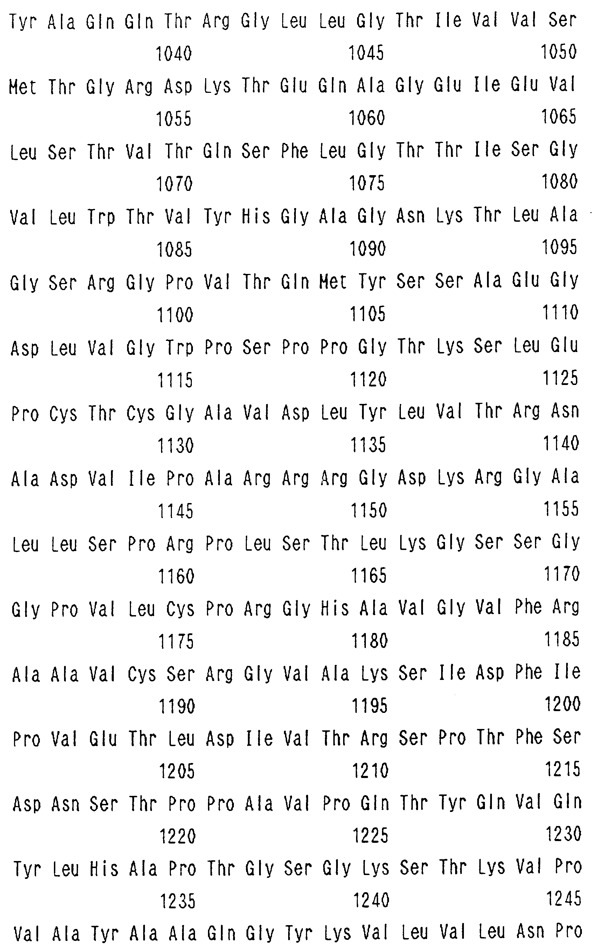

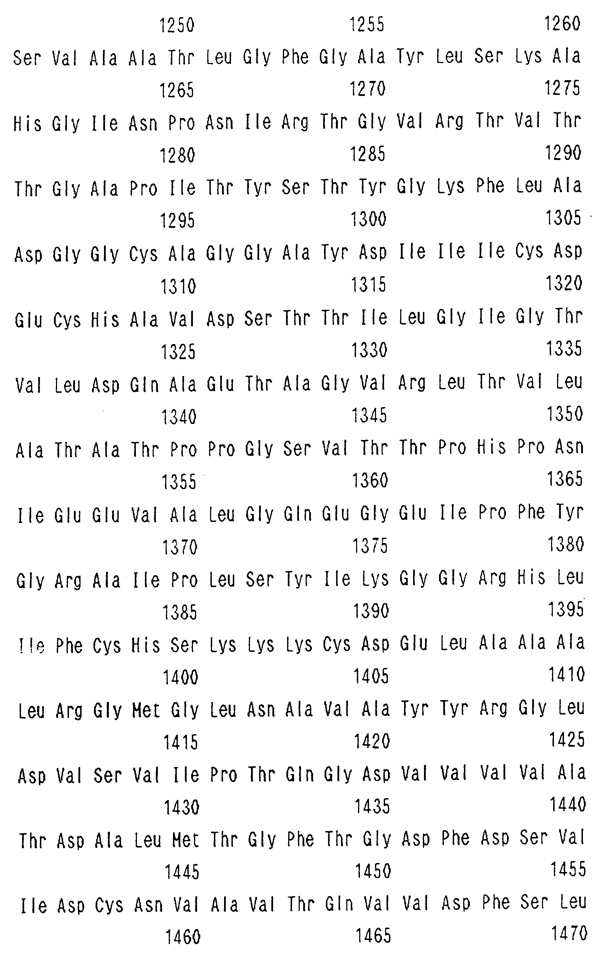

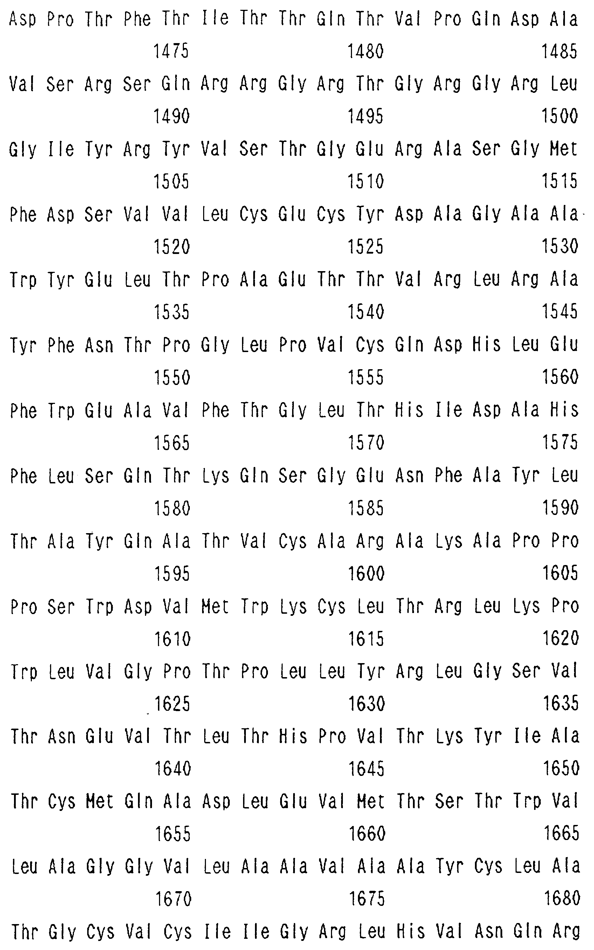

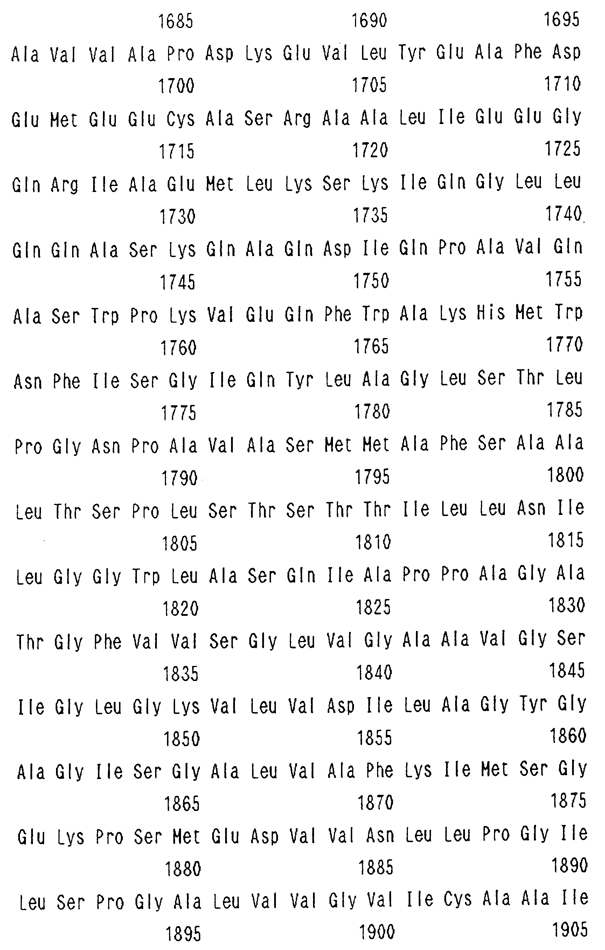

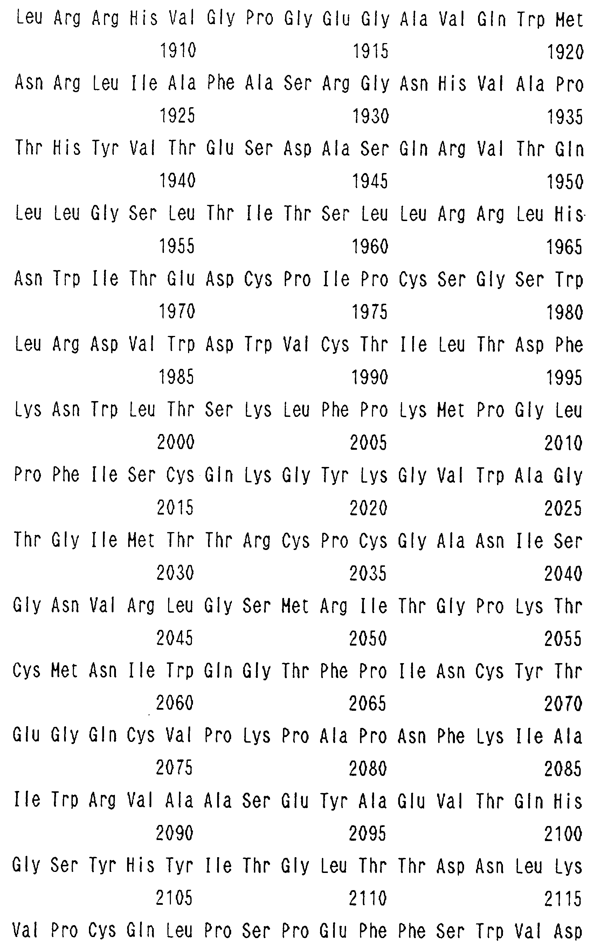

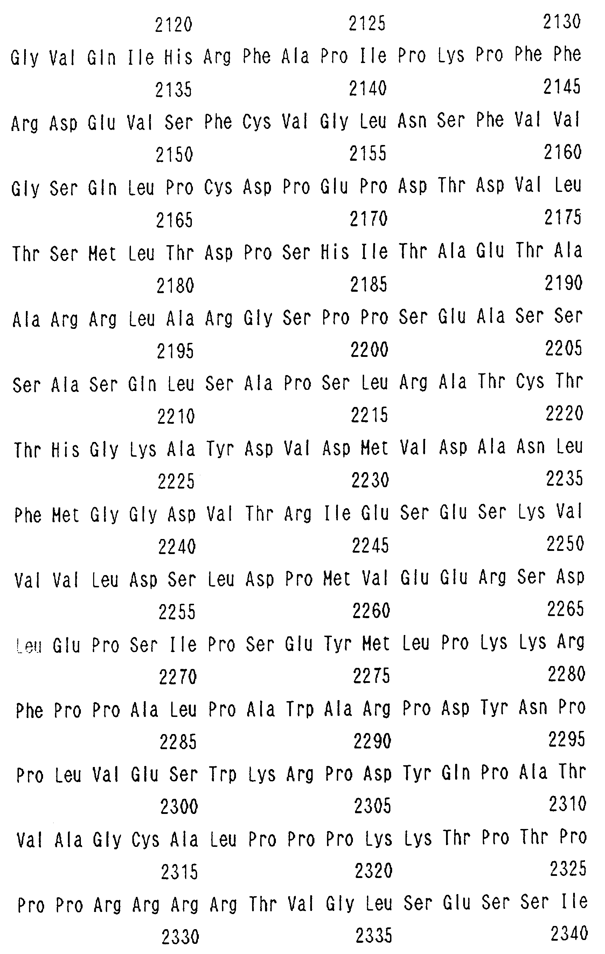

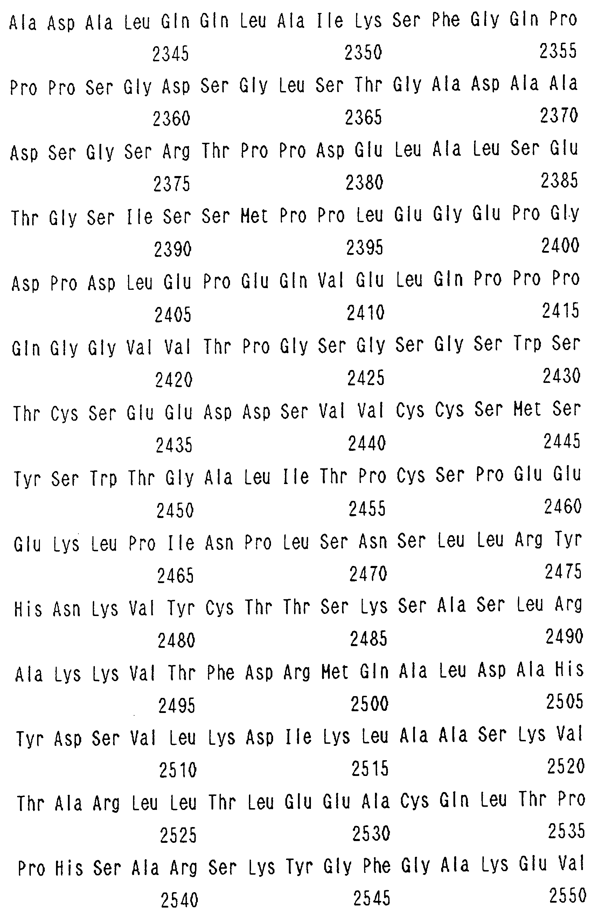

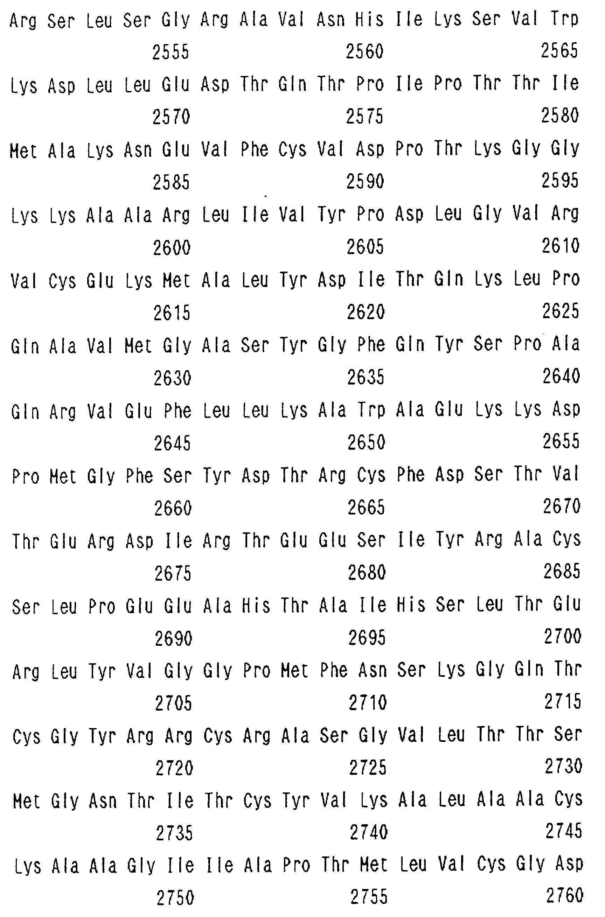

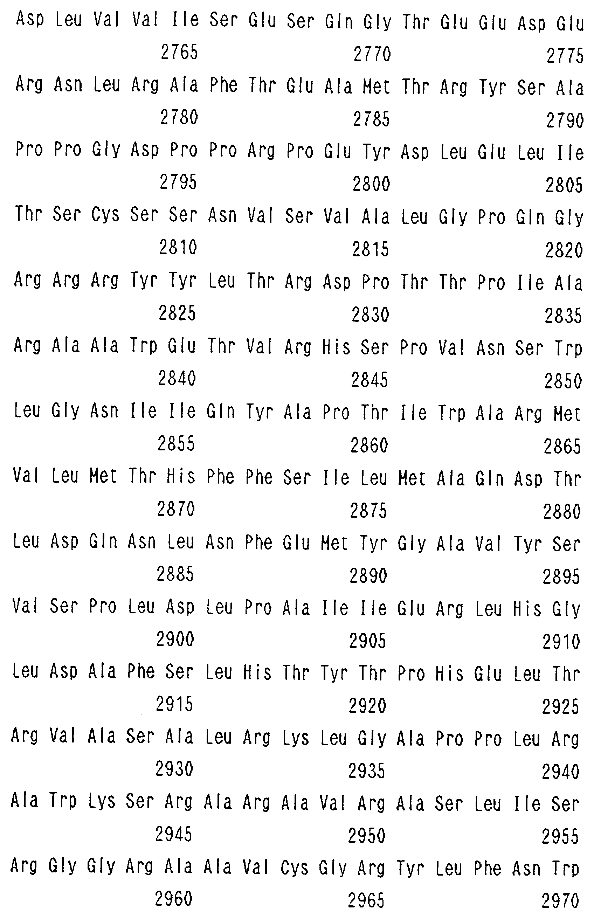

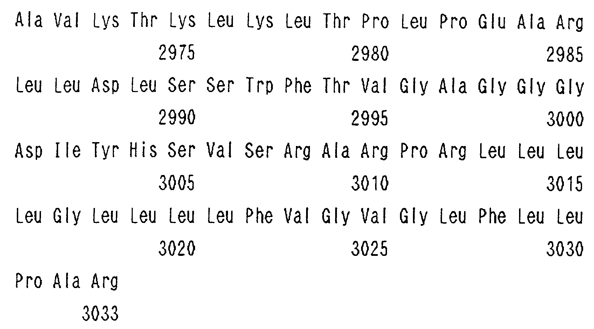

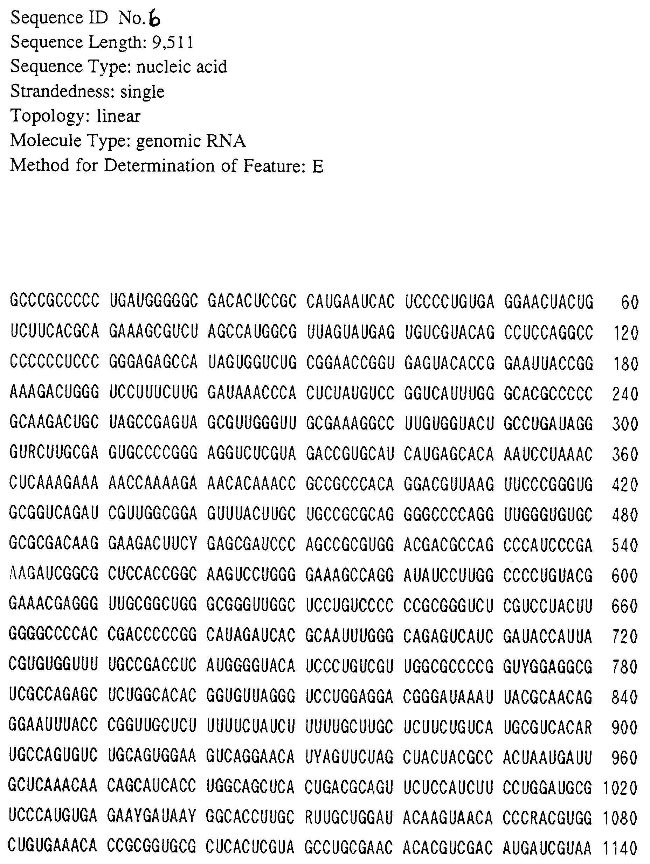

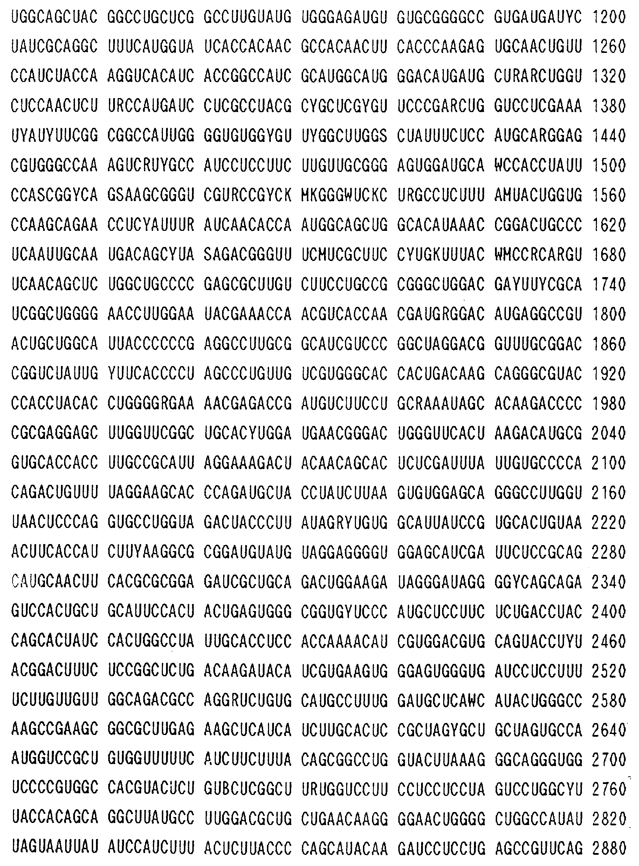

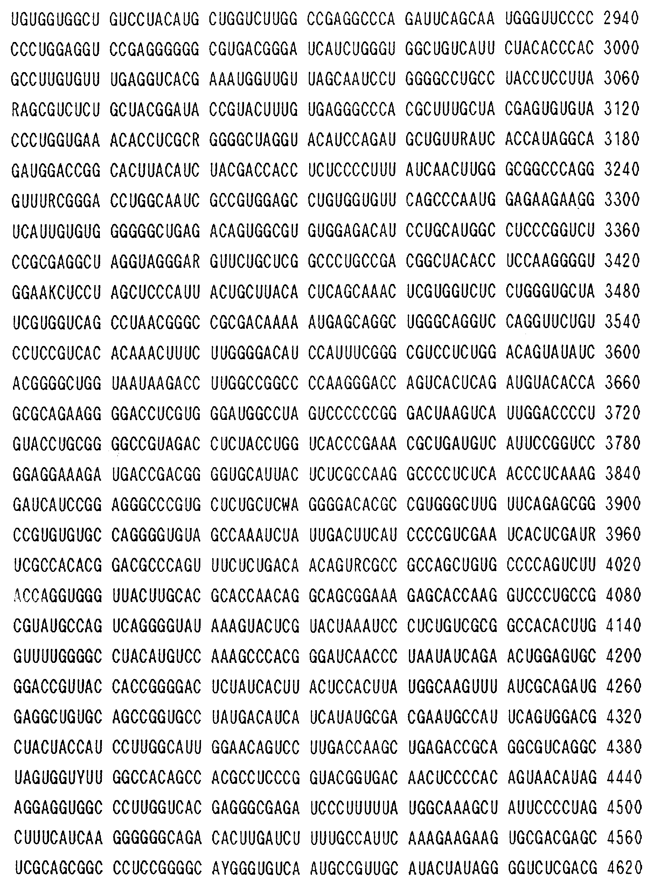

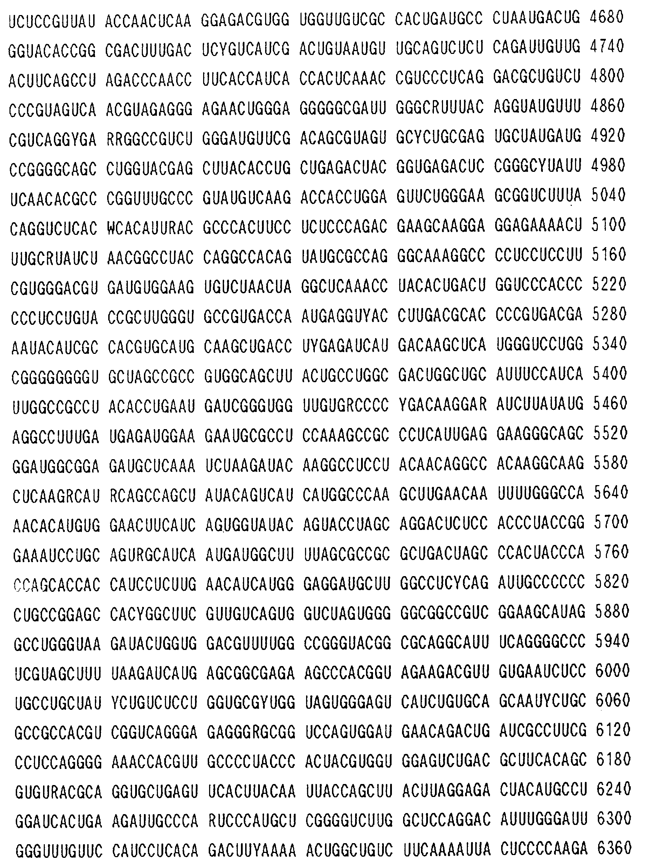

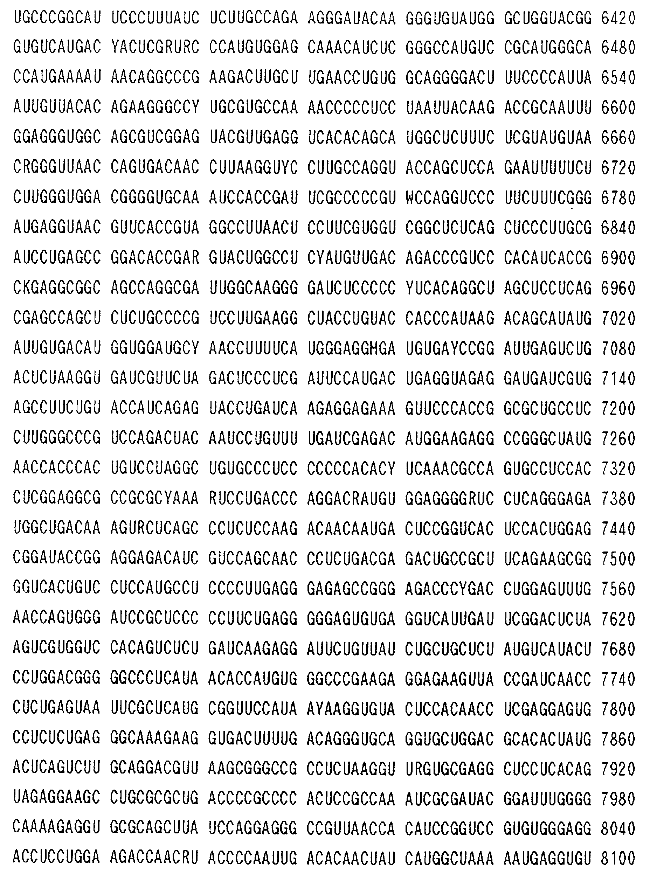

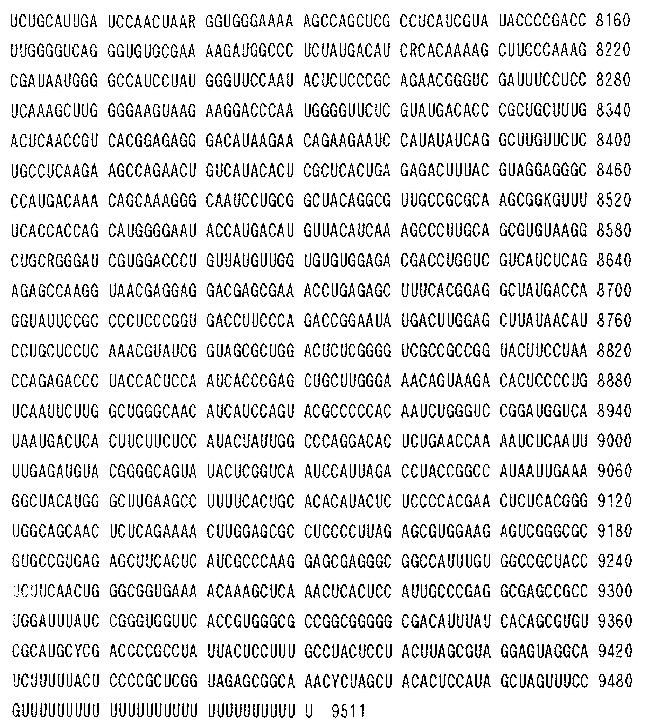

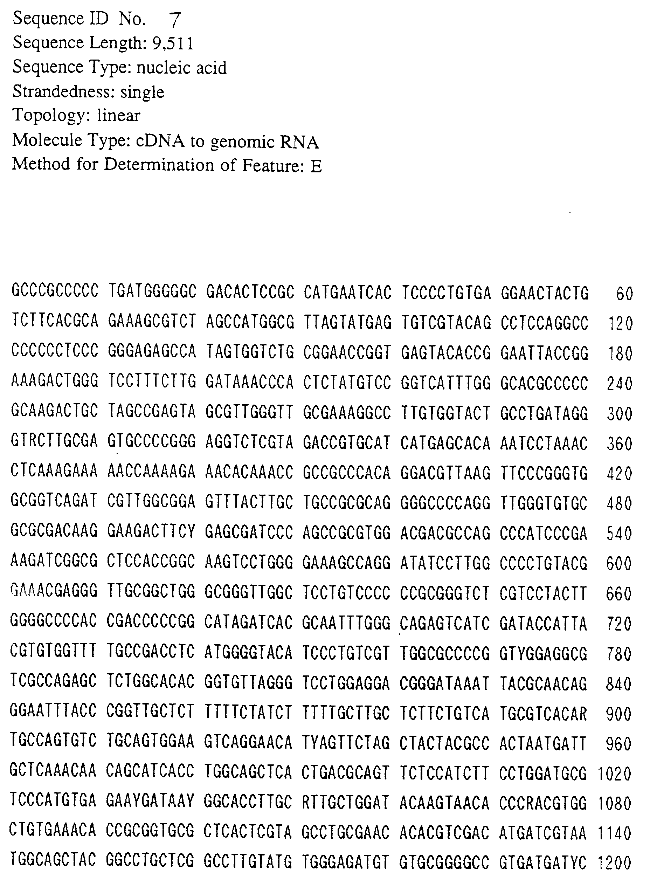

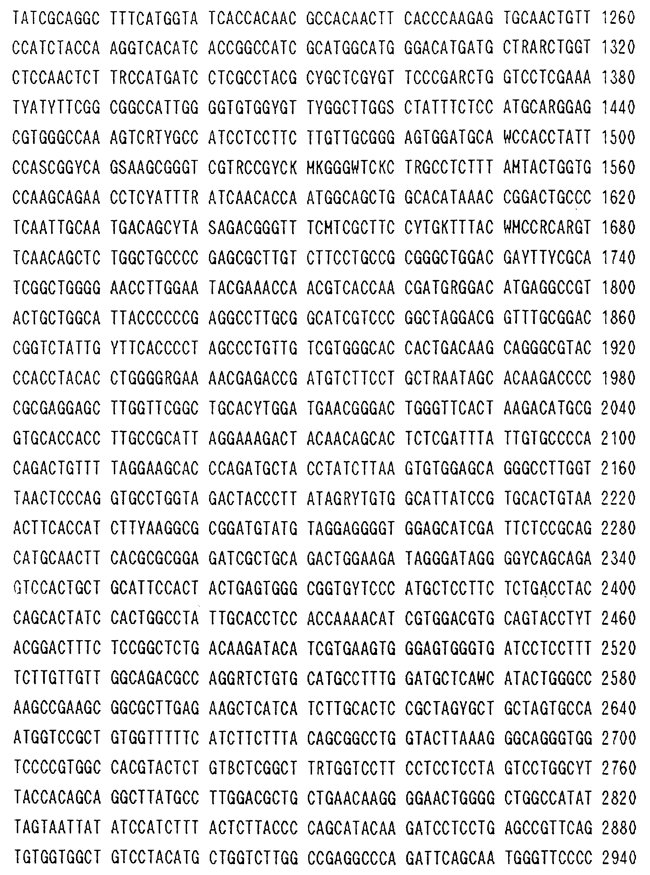

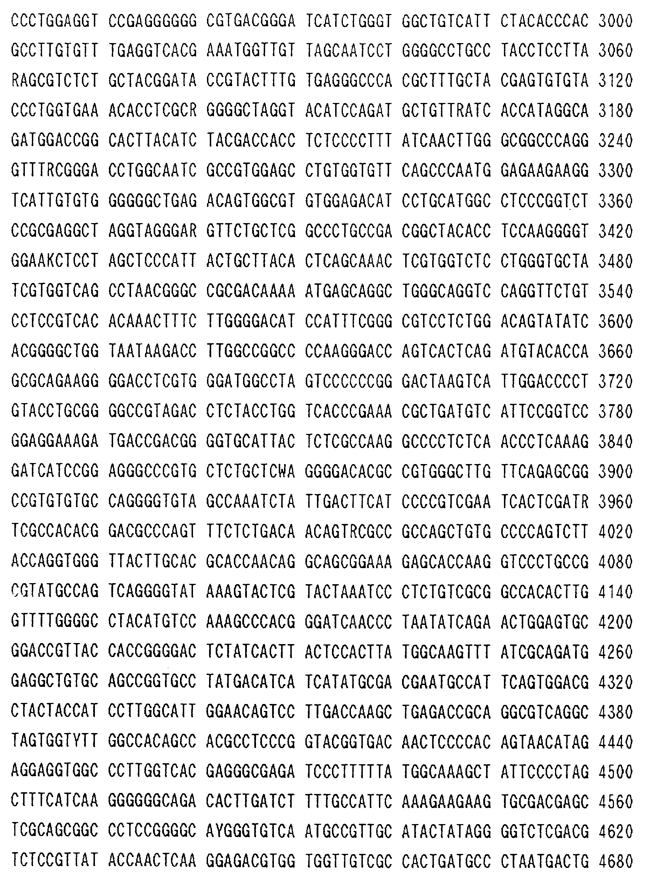

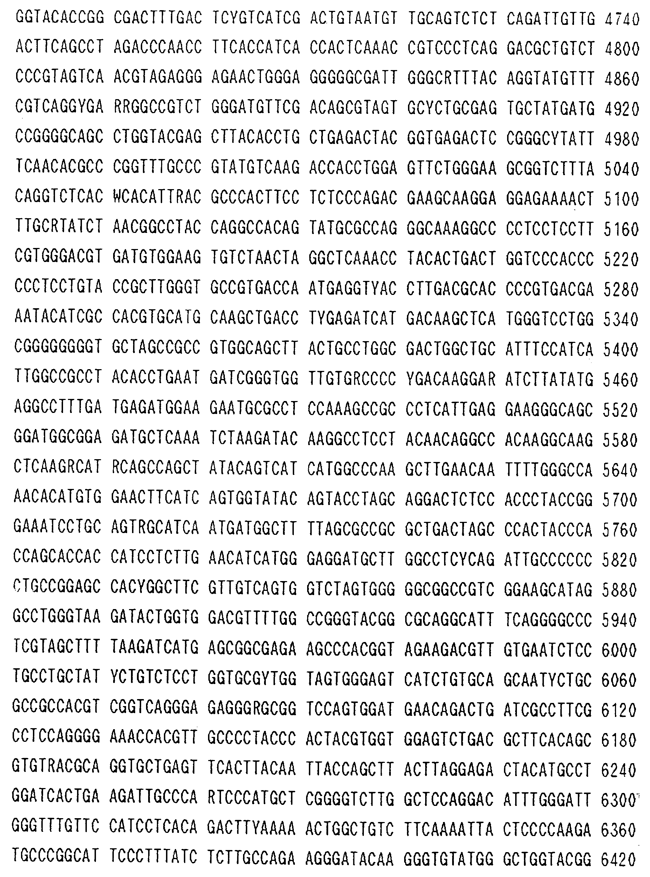

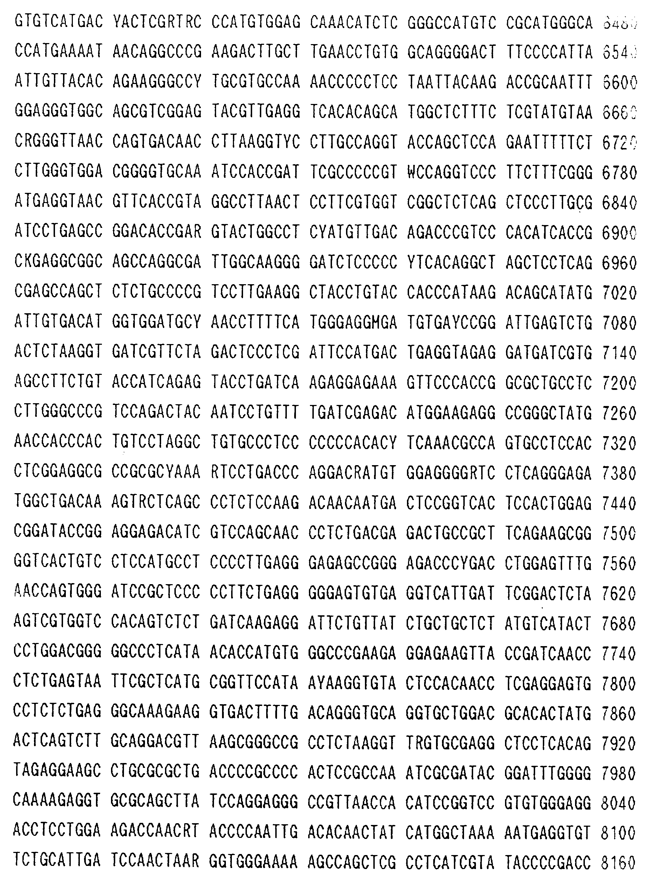

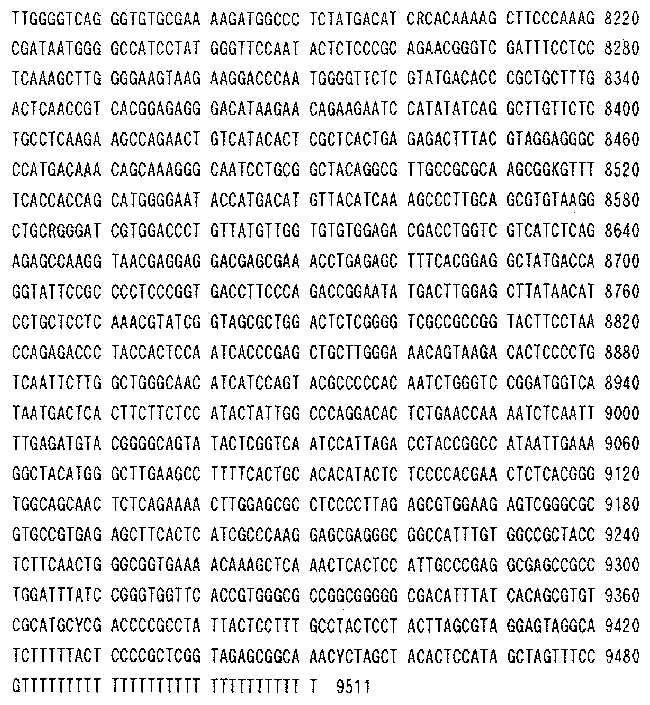

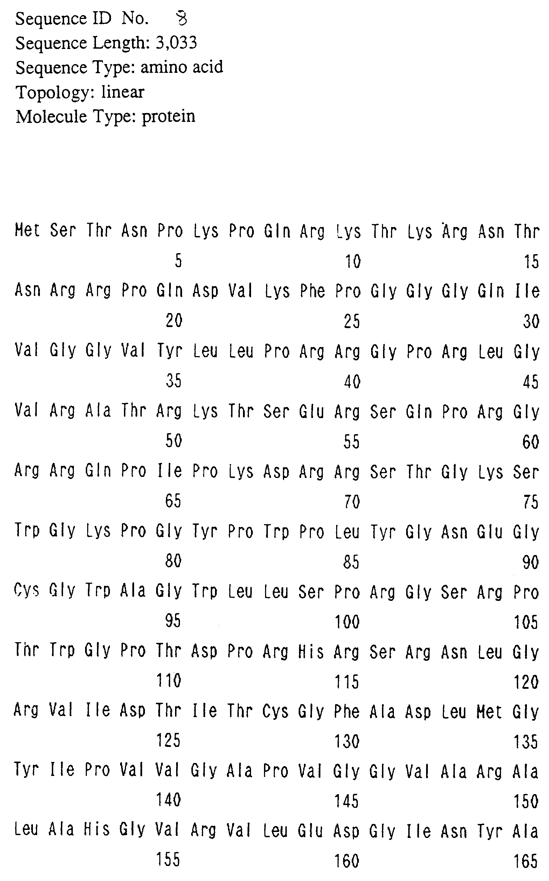

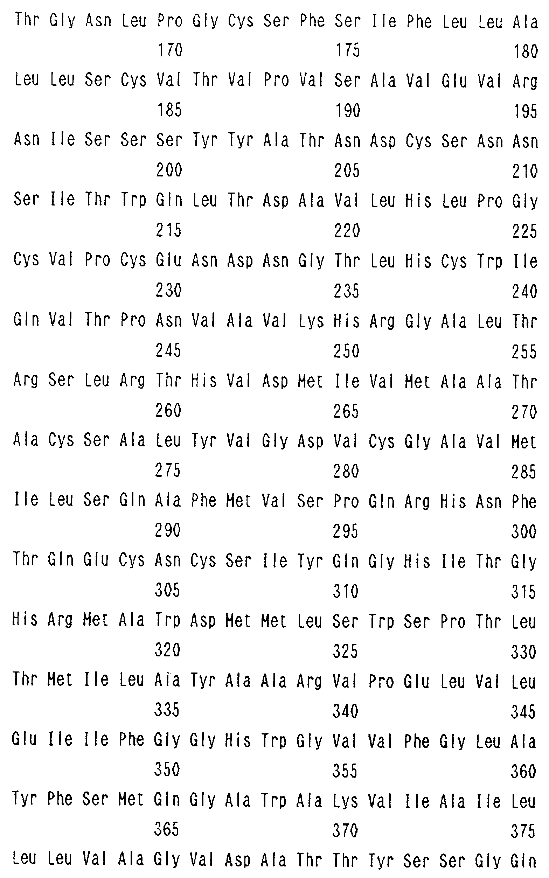

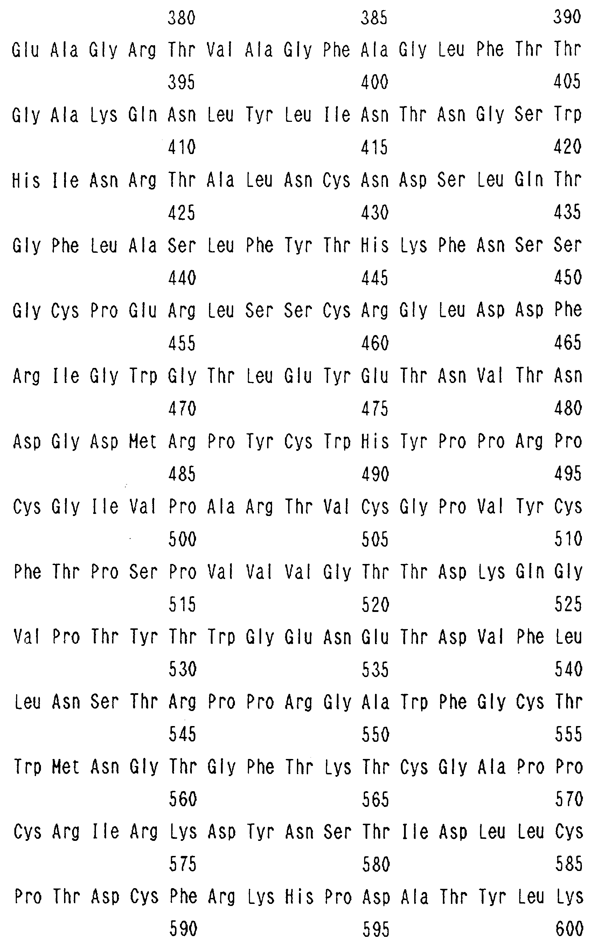

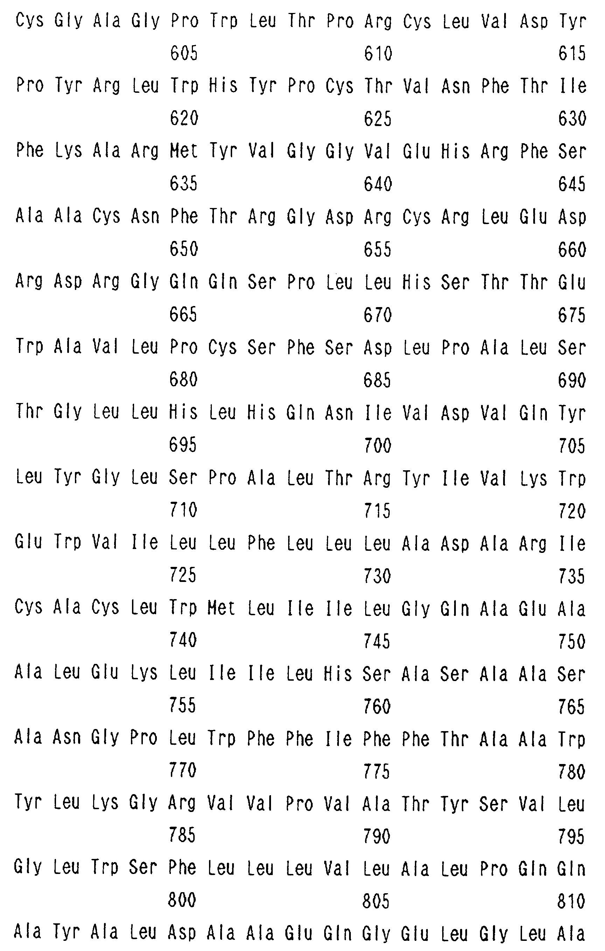

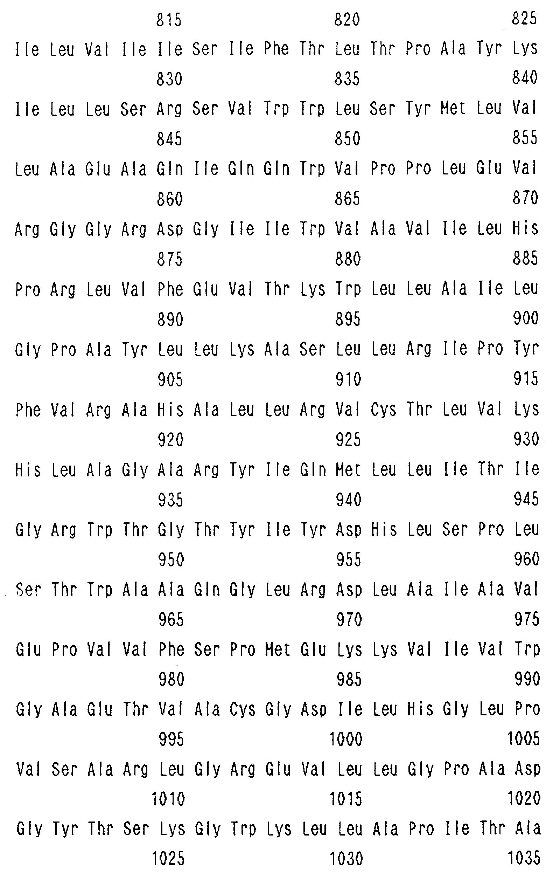

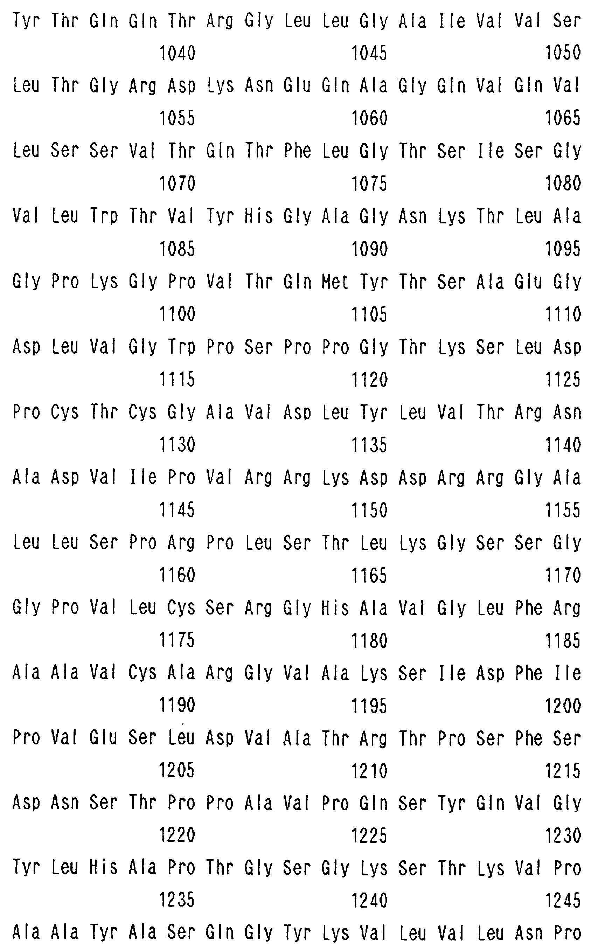

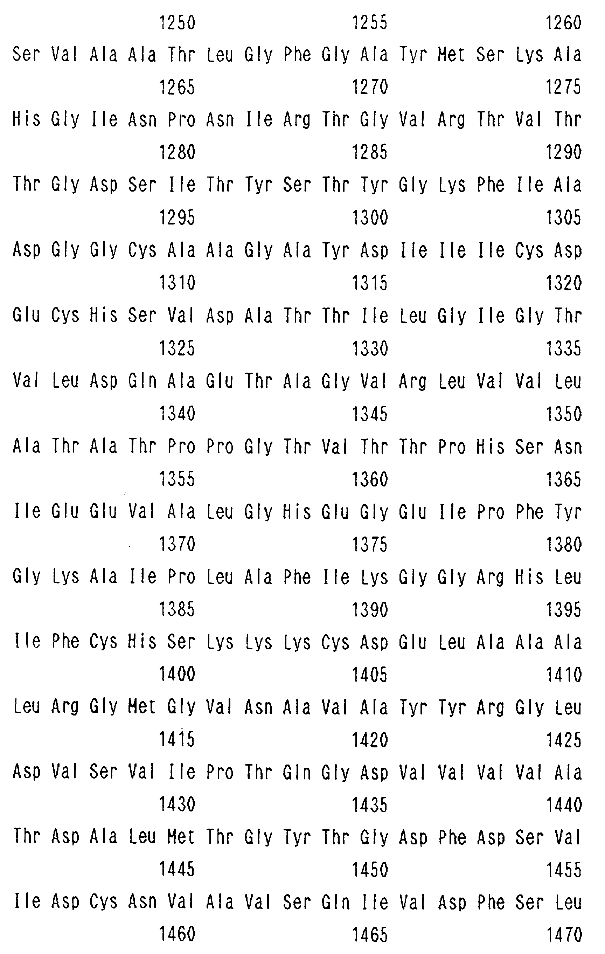

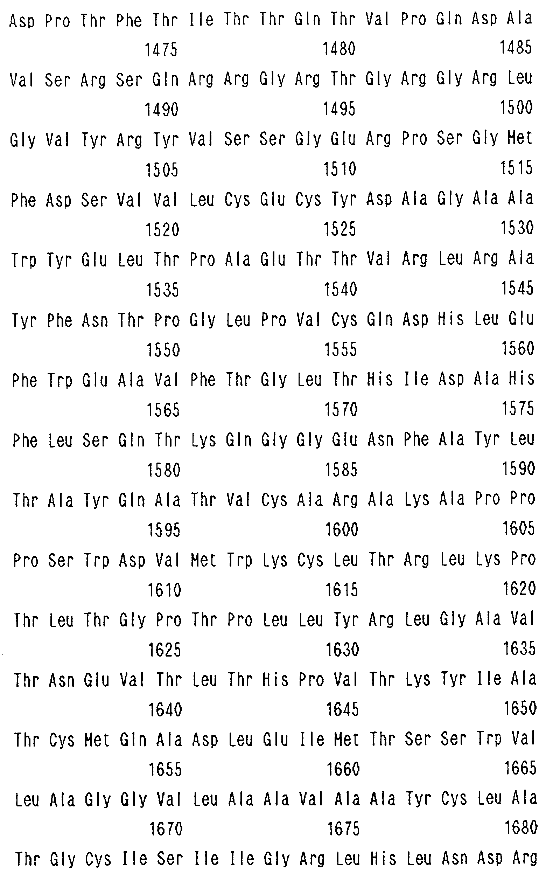

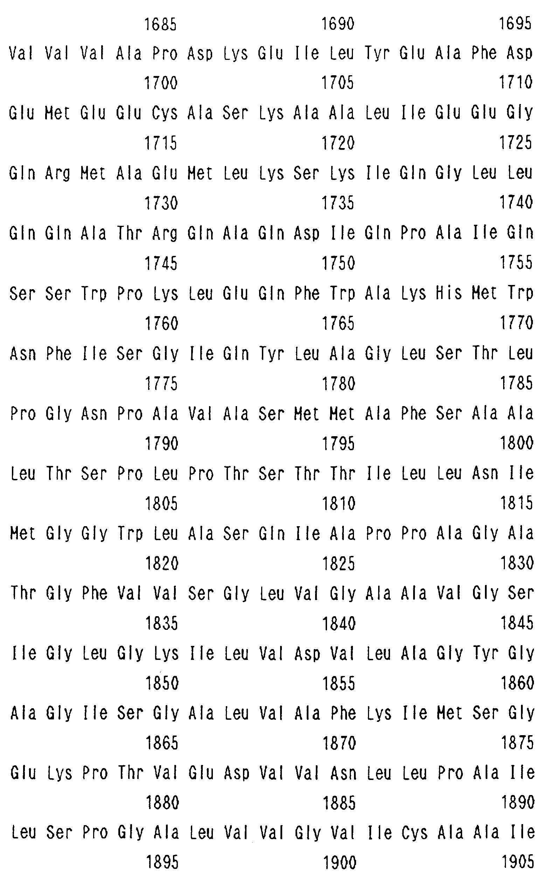

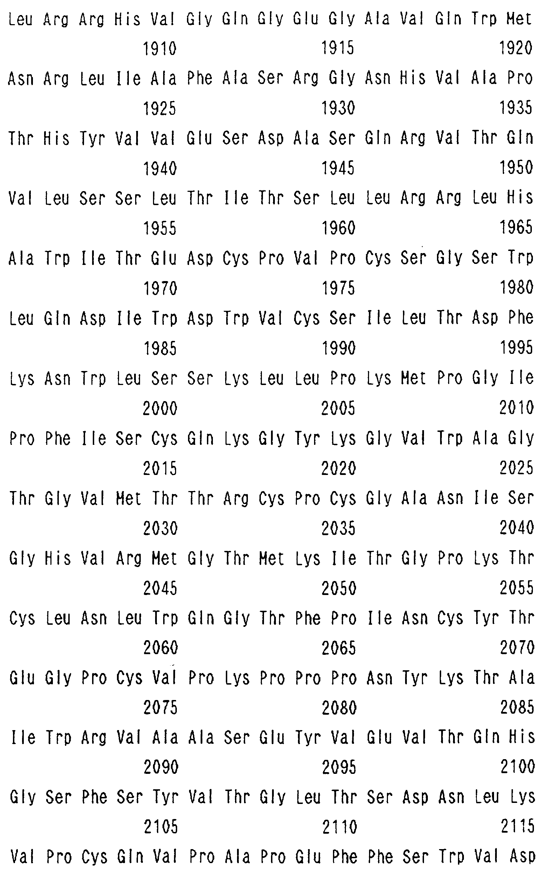

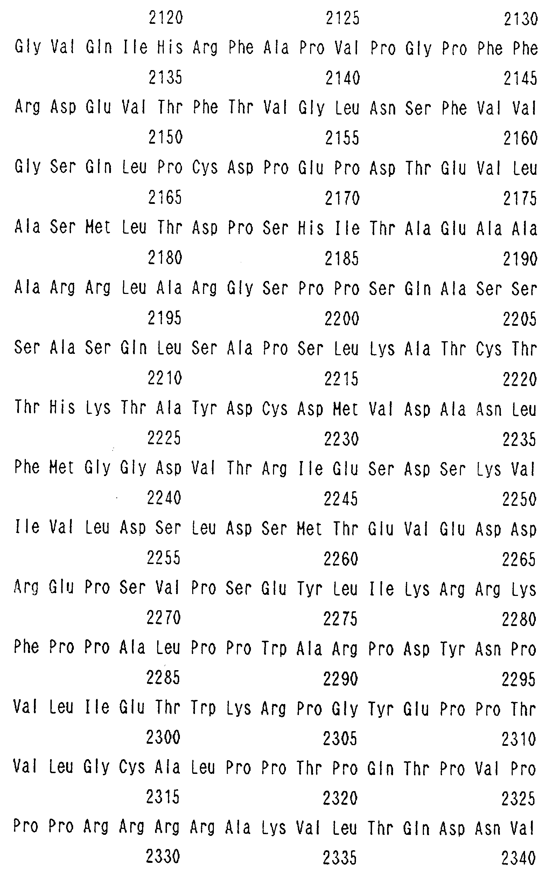

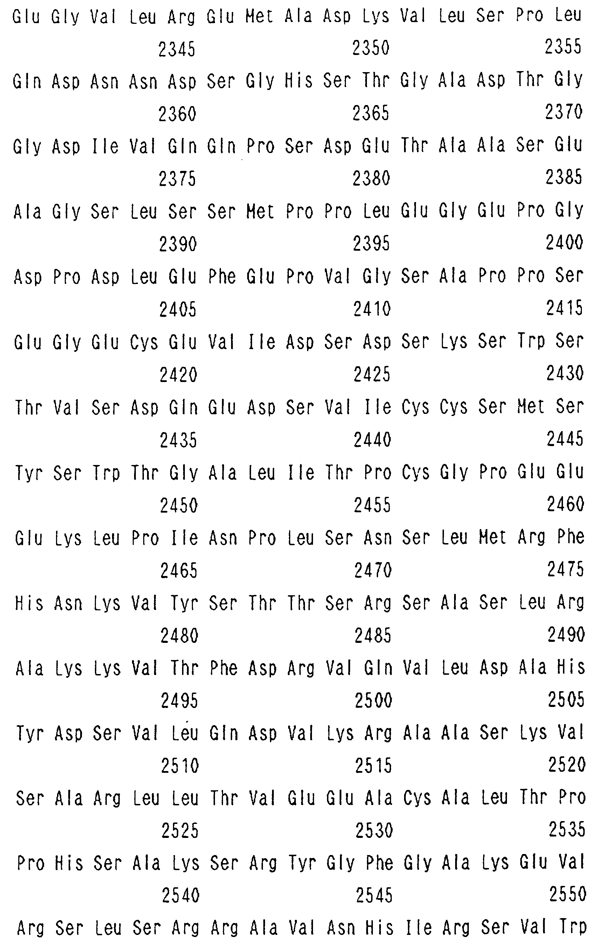

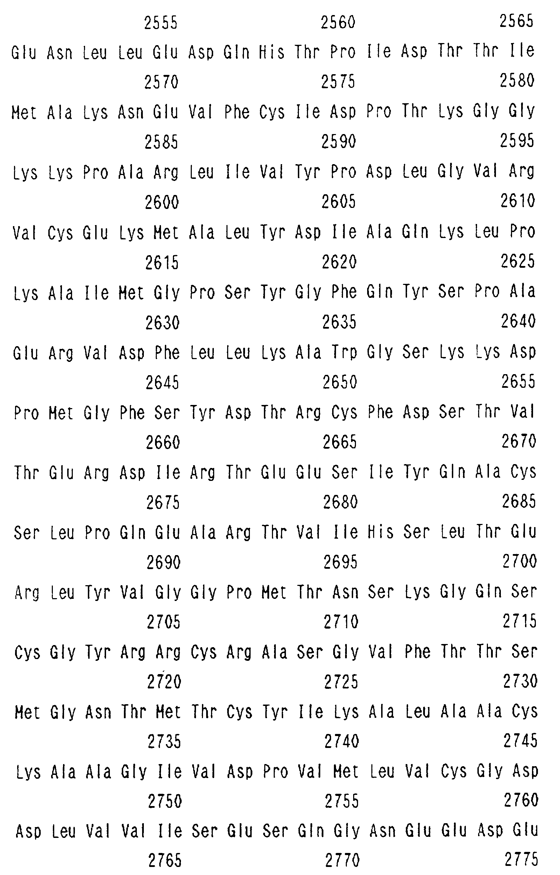

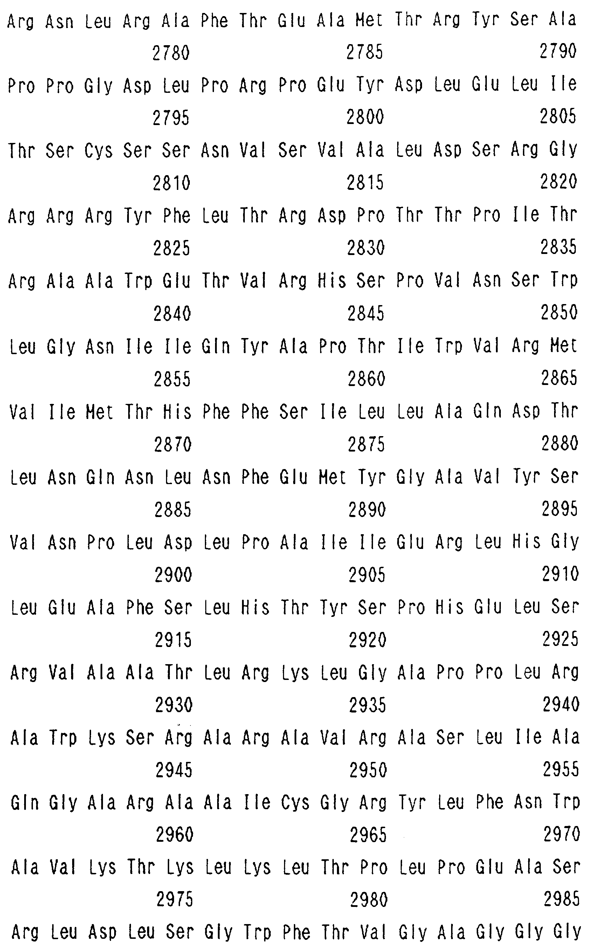

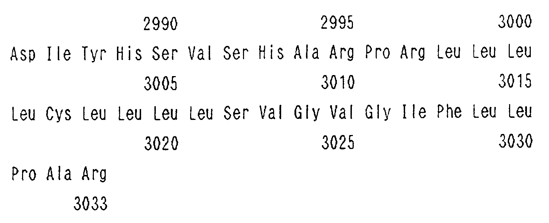

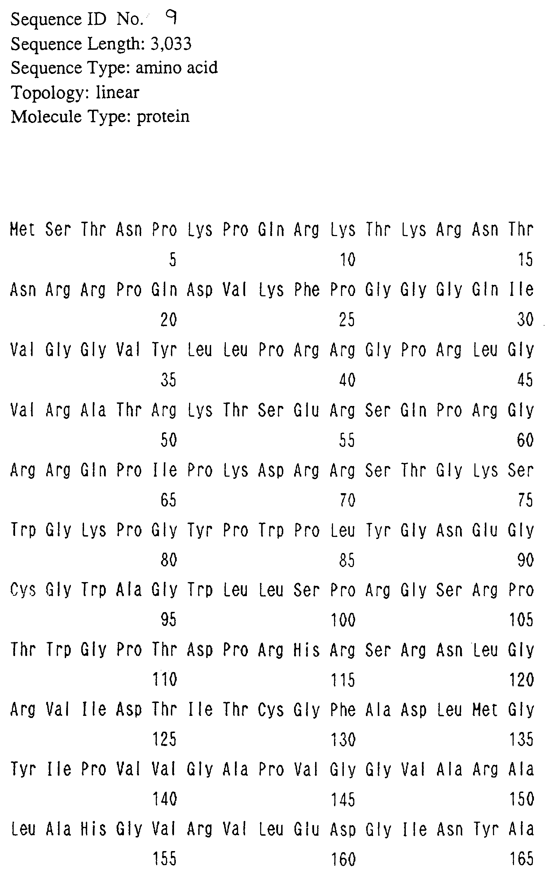

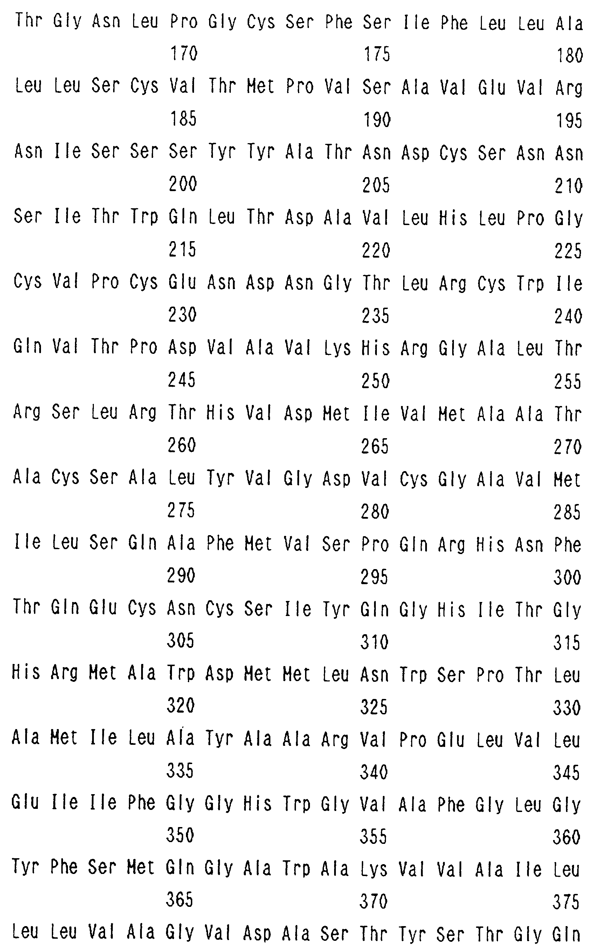

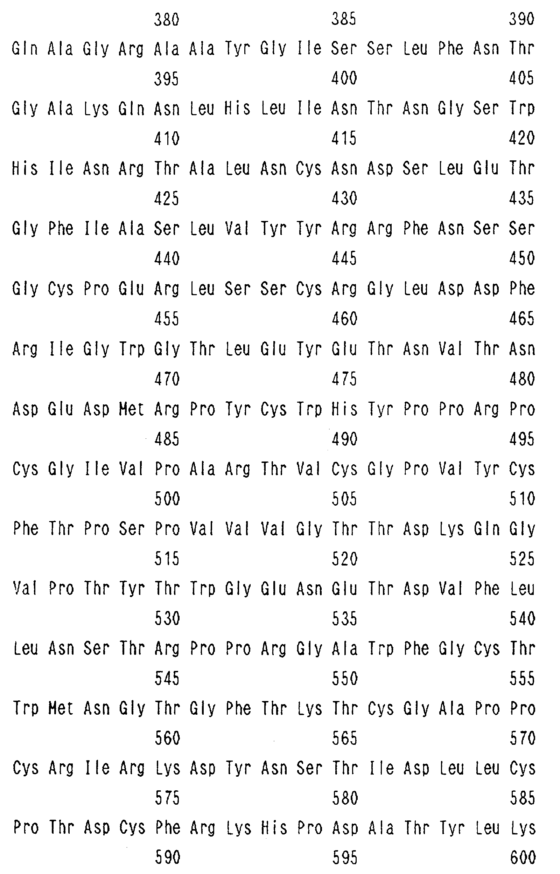

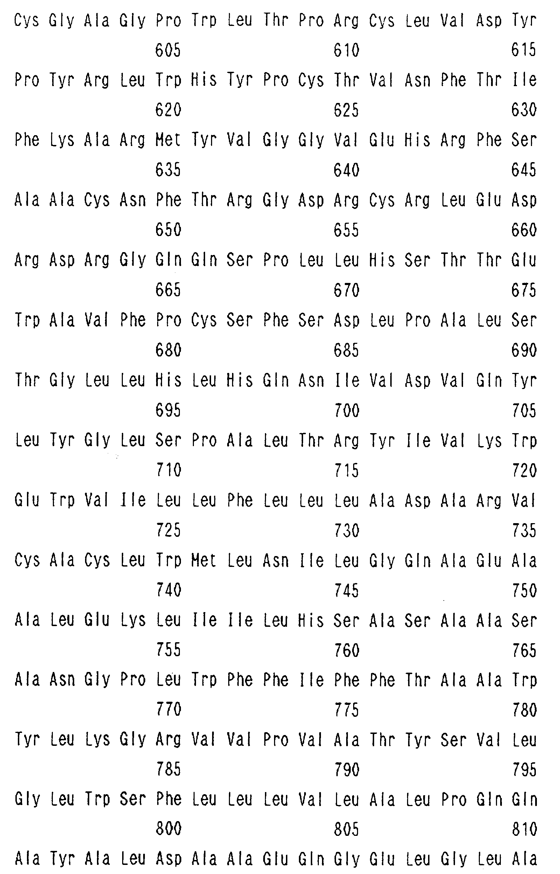

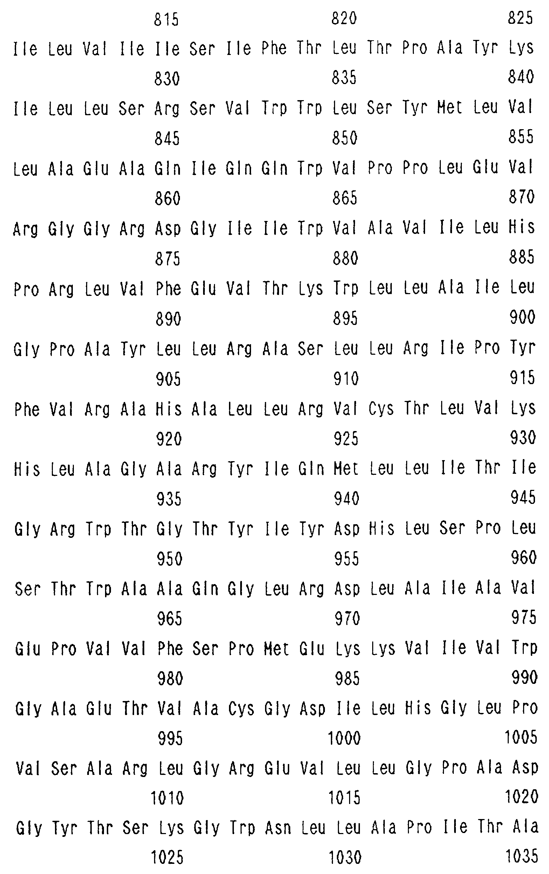

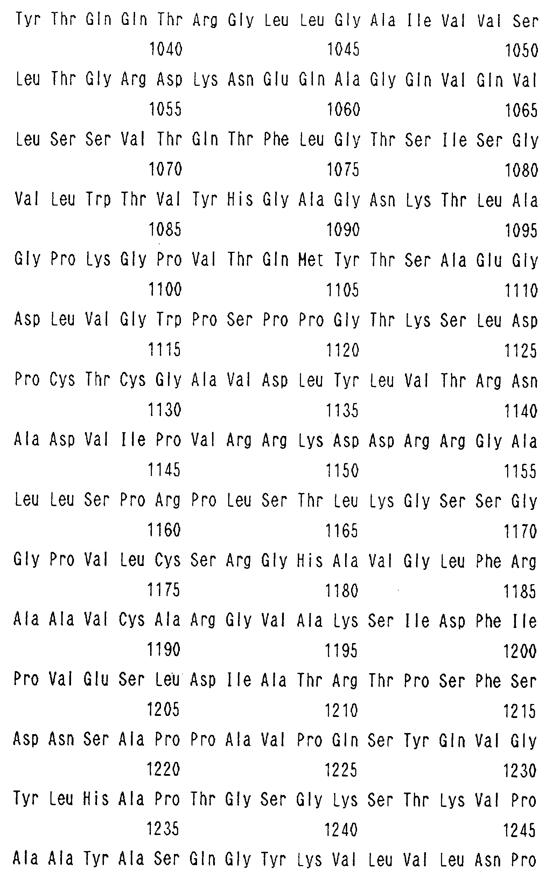

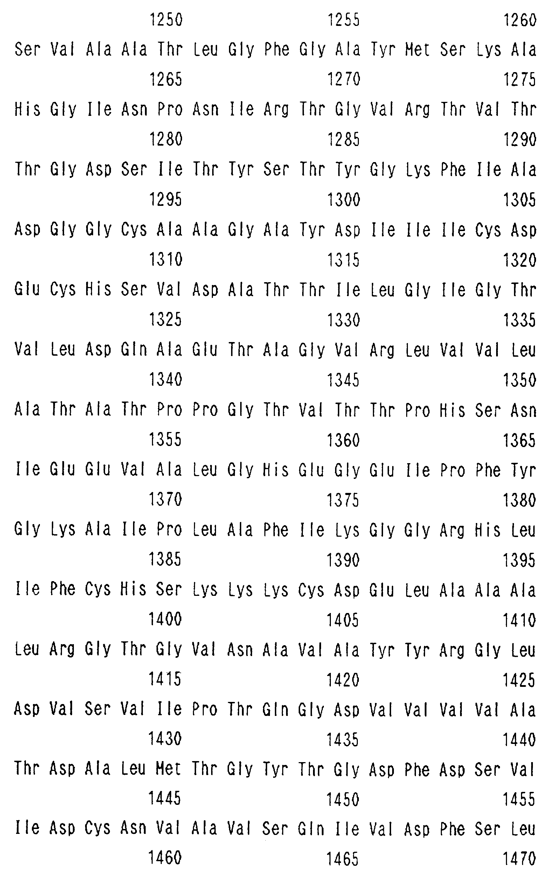

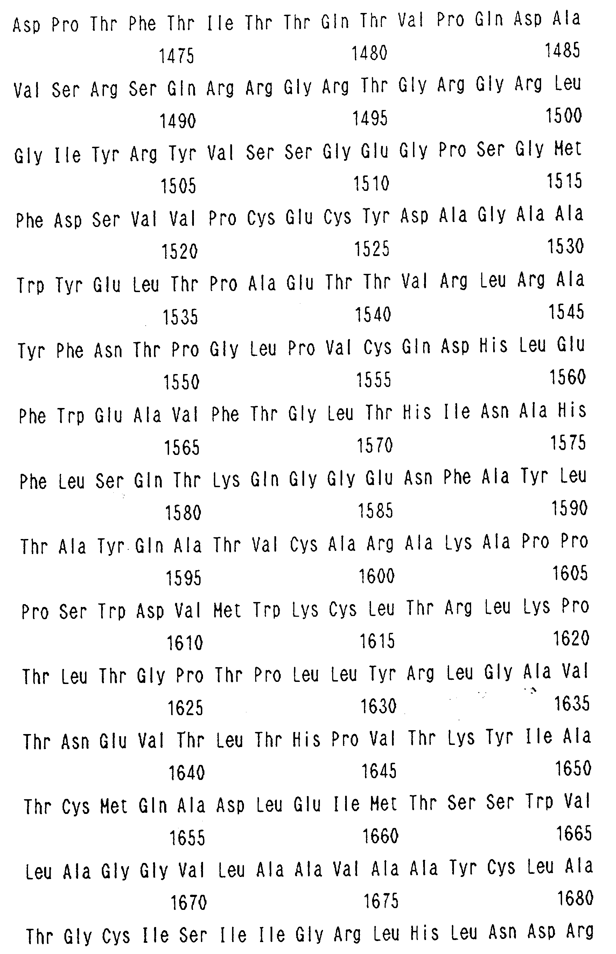

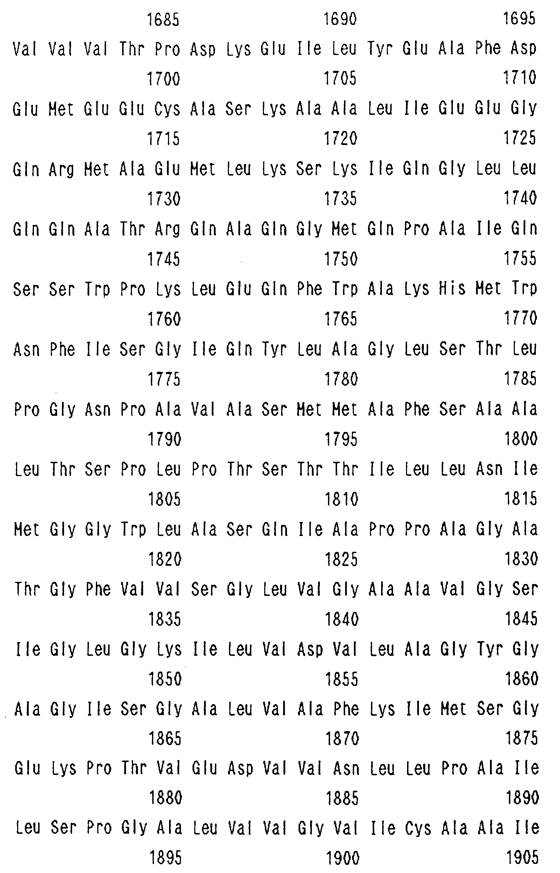

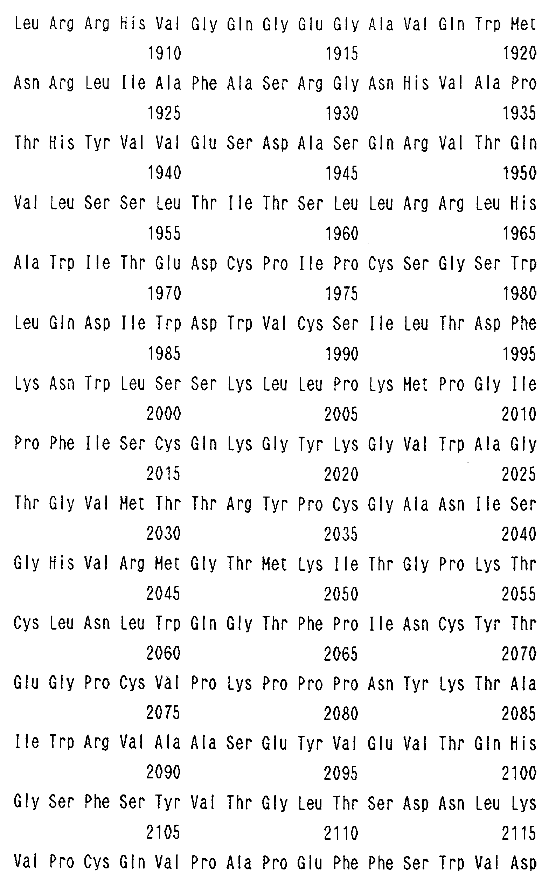

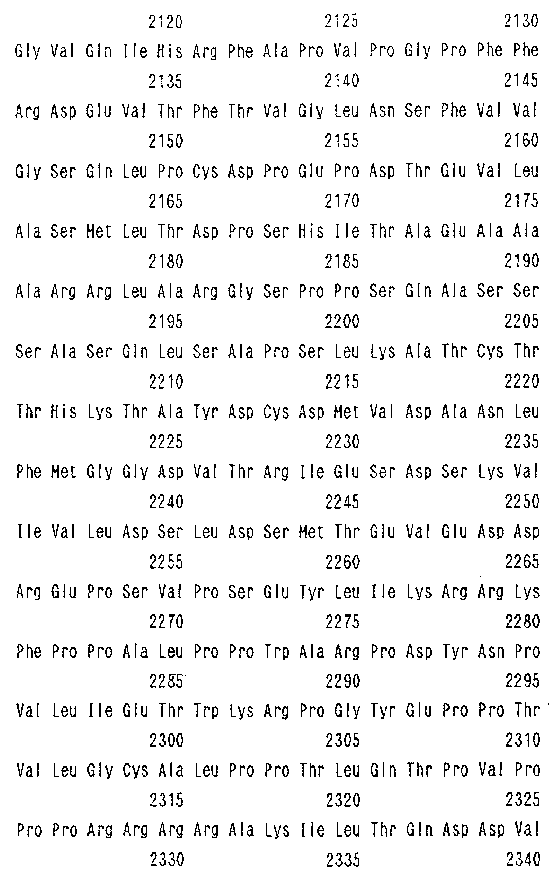

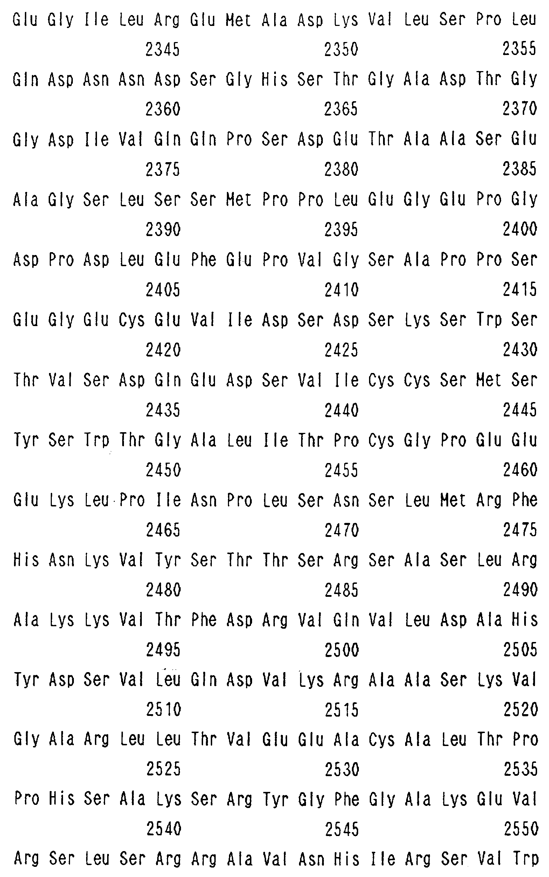

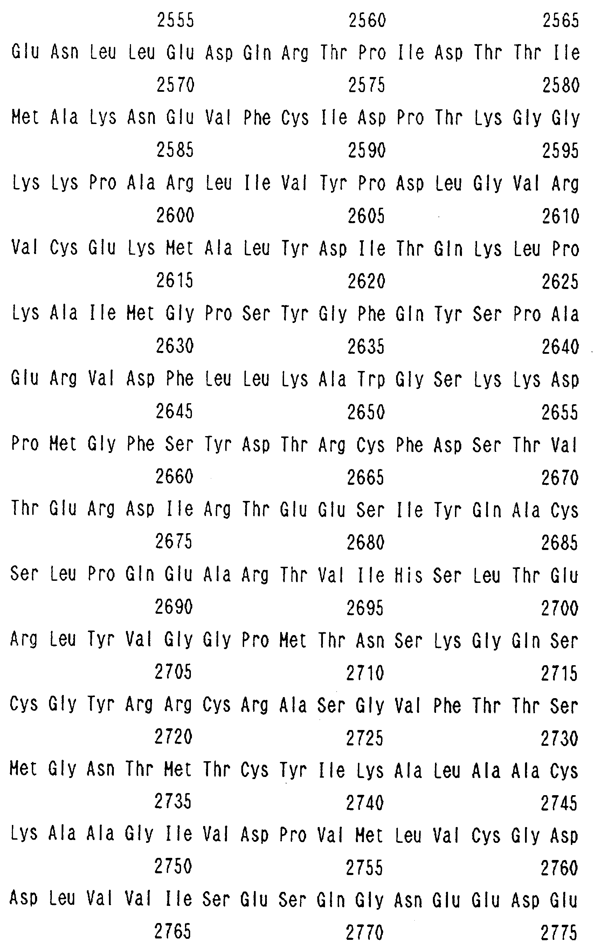

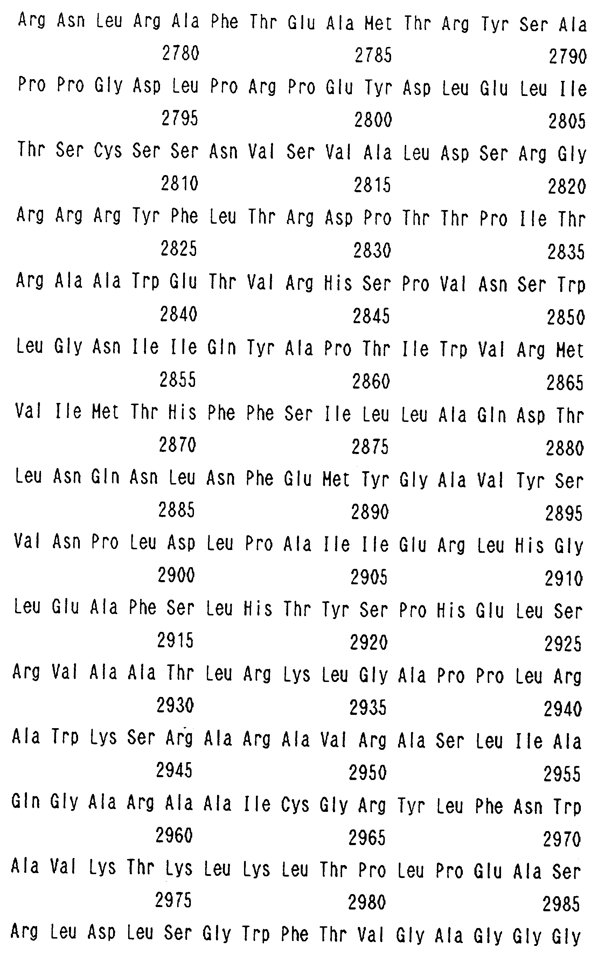

As shown in Figure 6, the nucleotide sequence of 5' terminus of HC-J8 genome (a region) was determined by analysis of clones obtained by amplification of HCV specific cDNA by PCR.

Single-stranded cDNA was synthesized using antisense primer #36 (5'-AACACTACTCGGCTAGCAGT-3') of nt246 to 265 in the same manner as (2) above, then it was added with dATP tail at its 3' terminus by terminal deoxynucleotidyl transferase, then amplified by one-sided PCR in two stages.

That is, in the first stage, antisense primer #48 (5'-GTTGATCCAAGAAAGGACCC-3') of nt188 to 207 was used with sense primer selected from non-specific primer #165 (5'-AAGGATCCGTCGACATCGATAATACG (A) ₁₇₋3') and #171 (5'-AAGGATCCGTCGACATCGATAATACG(T)₁₇-3') to amplify the dA-tailed cDNA by PCR for 35 cycles; and in the second stage, using the product of the first-stage PCR as a template, non-specific primer #166 (5' AAGGATCCGTCGACATCGAT -3') and antisense primer #109 (21-mer; 5'-ACCGGATCCGCAGACCACTAT -3') were added to initiate PCR for 30 cycles. The product of PCR was subcloned to M13 phage vector.

Thirteen independent clones (poly dT-tailed: C14951,C14952, C14953, C14958, C14960, C14968, C14971, C14972 and C14974; poly dA-tailed: C14987, C14996, C14999 and C15000) were obtained (each considered having complete length of 5' terminus), and the consensus sequence of nt1-139 of the respective clones was determined.

### (10) cDNA amplification of ORF region and 3' terminus by PCR

As shown in Figure 6, the nucleotide sequence of downstream from nt140 of HC-J8 genome was determined by analysis of clones obtained by amplification of HCV specific cDNA by PCR.

Single-stranded cDNAs to HC-J8 RNA were synthesized in the same manner as (2) above using antisense primers described below, then they were amplified by PCR using sense and antisense primers described below. Each product of PCR was subcloned to M13 phage vector, then consensus sequence of the respective clones of each region was determined.

The primers for cDNA synthesis and PCR amplification, and the numbers of obtained clones are shown below for each region. Alphabetical symbol of each amplified region corresponds to that in Figure 6. 
b region

nt45-847

Primer for cDNA synthesis: #122 (5'-AGGTTCCCTGTTGCATAATT-3')

Primer for PCR: sense:    #32A (5'-CTGTGAGGAACTACTGTCTT-3')

                antisense #122

Clones:         C15221,C15222,C15223

c region

nt732-1354

Primmer for cDNA synthesis:#54 (5'-ATCGCGTACGCCAGGATCAT-3')

Primer for PCR:  sense:    #50 (5'-GCCGATCTCATGGGGTACAT-3')

                 antisense:#54

Clones:          C15256,C15257,C15258

d region

nt1300-1879

Primer for cDNA synthesis: #199 (5'-GGGGTGAAACAATACACCGG-3')

Primer for PCR: sense:     #205 (5'-GGGACATGATGATCAACTGG-3')

                antisense: #199

Clones:         C14221,C14222,C14223
 
e region

nt1833-2518

Primer for cDNA synthesis: #146 (5'-AGTAGCATCATCCACAAGCA-3')

Primer for PCR: sense:     #150 (5'-ATCGTCTCGGCTAAGACGGT-3')

                antisense: #146

Clones:         C11535,C11540,C11566

f region

nt2433-3451

Primer for cDNA synthesis: #170 (5'-GCATAAGCAGTGATGGGGGC-3')

Primer for PCR: sense:     #160 (5'-CAGAACATCGTGGACGTGCA-3')

                antisense: #170

Clones:         C15348,C15349,C15356

g region

nt3404-4300

Primer for cDNA synthesis: #225 (5'-TCGCATATGATGATGTCATA-3')

Primer for PCR: sense:     #238 (5'-CTACACCTCCAAGGGGTGGA-3')

                antisense: #225

Clones:         C15701,C15702,C15703

h region

nt4221-5015

Primer for cDNA synthesis: #216 (5'-GTGGTCTAGACATACGGGCA-3')

Primer for PCR: sense:     #230 (5'-CCCATCACGTACTCCACATA-3')

                antisense: #216
 
Clones:          C15391,C15392,C15393

i region

nt4695-5062

Primer for cDNA synthesis:  #210 (5'-GCATCTATGTGTGTGAGGCC-3')

Primer for PCR: sense:      #209 (5'-TTCGACTCCGTGATCGACTG-3')

                antisense:  #210

Clones:          C14087,C14088,C14089

j region

nt5021-6169

Primer for cDNA synthesis: #162 (5'-TCCGACTCCGTCACGTAGTG-3')

Primer for PCR: sense:     #227 (5'-GTTCTGGGAAGCGGTCTTTA-3')

                antisense: #162

Clones:          C15421,C15422,C15423

k region

nt6027-6889

Primer for cDNA synthesis: #232 (5'-GATGGGTCTGTTAGCATGGA-3')

Primer for PCR: sense:     #242 (5'-TTGGTAGTGGGAGTCATCTG-3')

                antisense: #232

Clones:          C15733,C15734,C15735

l region

nt6834-7735

Primer for cDNA synthesis #239 (5'-ATCGGTAACTTCTCCTCTTC-3')
 
Primer for PCR: sense:    #241 (5'-CCTTGCGATCCTGAACCTGA-3')
                antisense:#239
Clones:         C15798,C15799,C15800

m region
nt7656-8630
Primer for cDNA synthesis: #222 (5'-GACCAGGTCGTCTCCACACA-3')
Primer for PCR: sense:     #229 (5'-GTCGTGTGCTGCTCCATGTC-3')
                antisense: #222
Clones:         C15376,C15378,C15381

n region
nt8325-9511
Primer for cDNA synthesis: #165
Primer for PCR: sense:     #80 (5'-GACACCCGCTGTTTTGACTC-3')
           non-specific:   #165
Clones:         C15270,C15271,C15272
From the analysis described above, full nucleotide sequence of cDNA to HC-J8 was determined as shown in sequence list 7, then full nucleotide sequence of HC-J8 genome RNA as shown in sequence list 6. Two amino acid sequences shown in sequence lists 8 and 9 represent those coded for by HC-J8 genome.

Utilizing known immunological techniques, it is possible to determine epitopes (e.g., from the core region, etc.) from the polypeptides of sequence lists 5, 8 and 9. Determination of such epitopes of the NANB hepatitis virus opens access to chemical synthesis of the peptide, manufacturing of the peptide by genetic engineering techniques, synthesis of the polynucleotides, manufacturing of the antibody, manufacturing of NANB hepatitis diagnostic reagents, and development of products such as NANB hepatitis vaccines.

According to the well-known method described by Merrifield, NAMB peptides can be synthesized. Furthermore, the polynucleotides in sequence lists 2-4 and 7 can be used to express polypeptides in host cells such as *Escherichia coli* by means of genetic engineering technique.

A detection system for antibody against NANB hepatitis virus can be developed using polyvinyl microtiter plates and the sandwich method. For example, 50µl of 5 µg/ml concentration of a NANB peptide can be dispensed in each well of the microtiter plates and incubated overnight at room temperature for consolidation. The microplate wells can be washed five times with physiological saline containing 0.05% Tween 20. For overcoating, 100 µl of NaCl buffer containing 30% (v/v) of calf serum and 0.05% Tween 20 (CS buffer) can be dispensed in each well and discarded after incubation for 30 minutes at room temperature.

For determination of NANB antibodies in samples, in the primary reaction, 50µl of the CS buffer containing 30% calf serum and 10 µl of a sample can be dispensed in each microplate well and incubated on a microplate vibrator for one hour at room temperature. After completion of the reaction, microplate wells can be washed five times in the same way as previously described.

In the secondary reaction, as labeled antibody 1 ng of horseradish peroxidase labeled anti-human IgG mouse monoclonal antibodies (Fab' fragment: 22G, Institute of Immunology Co., Ltd., Tokyo, Japan) dissolved in 50 µl of calf serum can be dispensed in each microplate well, and incubated on a microplate vibrator for one hour at room temperature. Wells can be washed five times in the same way. After addition of hydrogen peroxide (as substrate) and 50 µl of O-phenylendiamine solution (as color developer) in each well, and after incubation for 30 minutes at room temperature, 50 µl of 4M sulphuric acid can be dispensed in each well to stop further color development and for reading absorbance at 492 nm.

The cut-off level of this assay system can be set by measuring a number of donor samples with normal serum ALT (alanine aminotransferase) value of 34 Karmen unit or below and which tested negative for anti-HCV.

The present invention makes possible detection of NANB hepatitis virus infection which could not be detected by conventional determination methods, and provide NANB hepatitis detection kits capable of highly specific and sensitive detection at an early phase of infection.

These features allow accurate diagnosis of patients at an early stage of the disease and also help to remove at higher rate NANB hepatitis virus carrier bloods through screening test of donor bloods.

Polypeptides and their antibodies under this invention can be utilized for manufacture of vaccines and immunological pharmaceuticals, and structural gene of NANB hepatitis virus provides indispensable tools for detection of polypeptide antigens and antibodies.

Antigen-antibody complexes can be detected by methods known in this art. Specific monoclonal and polyclonal antibodies can be obtained by immunizing such animals as mice, guinea pigs, rabbits, goats and horses with NANB peptides (e.g., bearing NANB hepatitis antigenic epitope).

The present invention is based on studies on isolated virus genome of NANB hepatitis virus named HC-J6 and HC-J8, and is completed by clarification of the full sequence of the nucleotides. The invention makes possible highly specific detection of NANB hepatitis virus and provision of polypeptide, polyclonal antibody and monoclonal antibody to prepare the test system.

Further variations and modifications of the invention will become apparent to those skilled in the art from the foregoing and are intended to be encompassed by the claims appended hereto.

Japanese Priority Applications 287402/91 filed August 9, 1991 and 360441/91 filed on December 5, 1991 are relied on and incorporated by reference. U.S. patent applications serial no. 07/540,604 (filed June 19, 1990), 07/653,090 (filed February 8, 1991) , and 07/712,875 (filed June 11, 1991) are incorporated by reference in their entirety.

### Sequence list

- Sequence list 1:: whole nucleotides of HC-J6 genome RNA
- Sequence list 2:: N-9589 whole nucleotides of cDNA to HC-J6 genome RNA
- Sequence list 3:: J6-ø81 nucleotides of clone J6-ø81
- Sequence list 4:: J6-ø8 nucleotides of clone J6-ø8
- Sequence list 5:: P-J6-3033 whole amino acids of ORF of HC-J6 genome
- Sequence list 6:: whole nucleotides of HC-J8 genome RNA
- Sequence list 7:: whole nucleotides of cDNA to HC-J8 genome RNA
- Sequence list 8:: whole amino acids of a variation of ORF of HC-J8 genome
- Sequence list 9:: whole amino acids of a variation of ORF of HC-J8 genome

## Claims

1. Recombinant RNA of non-A, non-B hepatitis virus, strain HC-J6, comprising the nucleotide sequence of sequence list 1.

2. Recombinant cDNA of non-A, non-B hepatitis virus, strain HC-J6, comprising the nucleotide sequence of sequence list 2.

3. cDNA clone J6-ø81 comprising the nucleotide sequence of sequence list 3.

4. cDNA clone J6-ø8 comprising the nucleotide sequence of sequence list 4.

5. Amino acid sequence corresponding to recombinant cDNA of non-A, non-B hepatitis virus, strain HC-J6, comprising the amino acid sequence of sequence list 5.

6. Recombinant RNA of non-A, non-B hepatitis virus, strain HC-J8, comprising the nucleotide sequence of sequence list 6.

7. Recombinant cDNA of non-A, non-B hepatitis virus, strain HC-J8, comprising the nucleotide sequence of sequence list 7.

8. Amino acid sequence corresponding to recombinant cDNA of non-A, non-B hepatitis virus, strain HC-J8, comprising the amino acid sequence of sequence list 8.

9. Amino acid sequence corresponding to recombinant cDNA of non-A, non-B hepatitis virus, strain HC-J8, comprising the amino acid sequence of sequence list 9.

10. A non-A, non-B hepatitis diagnostic test kit for analyzing samples for the presence of antibodies directed against a non-A, non-B hepatitis antigen, comprising an antigen attached to a solid substrate and labeled anti-human immunoglobulin; wherein said antigen is an antigen selected from the antigens contained in sequence lists 5, 8 or 9.

11. A method of detecting antibodies directed against a non-A, non-B hepatitis antigen in a sample, said method comprising:
(a) reacting said sample with an antigen selected from the antigens contained in sequence lists 5, 8 or 9 to form antigen-antibody complexes; and
(b) detecting said antigen-antibody complexes.

12. A non-A, non-B hepatitis specific monoclonal or polyclonal antibody reactive with an antigen, said antigen is an antigen selected from the antigens contained in sequence lists 5, 8 or 9.

13. A method of detecting non-A, non-B hepatitis antigen in a sample, said method comprising:
(a) reacting said sample with the non-A, non-B hepatitis monoclonal or polyclonal antibody according to claim 12 to form antigen-antibody complexes; and
(b) detecting said antigen-antibody complexes.
